(19) Europäisches Patentamt
European Patent Office
Office européen des brevets

(11) Veröffentlichungsnummer: **0 116 262**
**B1**

(12) # EUROPÄISCHE PATENTSCHRIFT

(45) Veröffentlichungstag der Patentschrift: **27.12.89**

(21) Anmeldenummer: **83810632.6**

(22) Anmeldetag: **30.12.83**

(51) Int. Cl.⁴: **C 07 D 249/08**, C 07 D 233/60, A 01 N 43/64, A 01 N 43/50 // C07D405/06

(54) **Fluorazolyl-propanol-Derivate als Mikrobizide und wuchsregulierende Mittel.**

(30) Priorität: **10.01.83 CH 112/83**

(43) Veröffentlichungstag der Anmeldung: **22.08.84 Patentblatt 84/34**

(45) Bekanntmachung des Hinweises auf die Patenterteilung: **27.12.89 Patentblatt 89/52**

(84) Benannte Vertragsstaaten: **AT BE CH DE FR GB IT LI NL SE**

(56) Entgegenhaltungen:
**EP-A-0 031 029**
**EP-A-0 040 345**

(73) Patentinhaber: **CIBA- GEIGY AG, Klybeckstrasse 141, CH- 4002 Basel (CH)**

(72) Erfinder: **Müller, Urs, Dr., Schluchtstrasse 15, CH- 4142 Münchenstein (CH)**
Erfinder: **Rempfler, Hermann, Dr., Brücklismattstrasse 16, CH- 4107 Ettingen (CH)**
Erfinder: **Tobler, Hans, Dr., Baselmattweg 157, CH- 4123 Allschwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

EP 0 116 262 B1

## Beschreibung

Die vorliegende Erfindung betrifft neue, substituierte Fluorazolylpropan-Derivate sowie deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen. Weiter betrifft die Erfindung als Zwischenprodukte hergestellte Fluorazolylmethyloxirane und Fluorazolylketone.

Die erfindungsgemässen Fluorazolylpropan-Derivate haben die Formel I

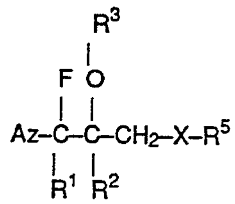

(I)

worin

Az für Imidazolyl oder 1,2,4-Triazolyl steht,
$R^1$ für Wasserstoff, für gegebenenfalls durch Halogen oder Cyan substituiertes $C_1$-$C_6$-Alkyl oder für gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl steht,
$R^2$ gegebenenfalls durch $C_1$-$C_3$-Alkoxy, Phenyl oder $C_1$-$C_3$-Phenylalkyl substituiertes $C_1$-$C_{10}$-Alkyl bedeutet, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,
$R^3$ -für Wasserstoff, $C_1$-$C_6$-Alkyl, $C_1$-$C_6$-Alkylcarbonyl oder gegebenenfalls durch Halogen, $C_1$-$C_3$-Alkoxy, $C_1$-$C_3$-Alkyl, $C_1$ -$C_3$-Halogenalkyl, Cyan, Carboxyl oder $C_1$-$C_6$-Alkoxycarbonyl substituiertes $C_1$-$C_3$-Phenylalkyl steht,
$R^5$ für einen unsubstituierten oder ein- oder mehrfach substituierten Rest steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, wobei die Substituenten aus der Reihe Halogen, Cyano, $C_1$-$C_3$-Alkyl, $C_1$-$C_5$-Alkoxy, $C_1$-$C_5$-Haloalkyl, $C_1$-$C_3$-Alkylthio, $C_1$-$C_3$-Haloalkyl, $C_1$-$C_3$-Haloalkylthio, Nitro und/oder Rhodano ausgewählt sind; und Sauerstoff oder Schwefel bedeuten; unter Einschluß der Säureadditionssalze, quaternären Azoliumsalze und Metallkomplexe.

Der Ausdruck Azolyl kennzeichnet einen fünfgliedrigen heterocyclischen Fünfring mit Stickstoff als Heteroatom und mit aromatischem Charakter. Typische Vertreter sind 1H-1,2,4-Triazol,4H-1,2,4-Triazol und 1H-Imidazol. Unter dem Begriff Alkyl selbst oder als Bestandteil eines anderen Substituenten, wie Alkoxy, Alkylthio, Halogenalkyl, Aralkyl oder Alkylcarbonyl sind je nach Zahl der angegebenen Kohlenstoffatome beispielsweise folgende Gruppen zu verstehen: Methyl, Äthyl, Propyl, Butyl, Pentyl, Hexyl, Heptyl, Octyl, Nonyl oder Decyl, sowie ihre Isomeren, wie z. B. Isopropyl, Isobutyl, sec.-Butyl, tert.-Butyl, Isopentyl usw.. Halogenalkyl steht für einen einfach bis perhalogenierten Alkylsubstituenten, wie z. B. $CHCl_2$, $CHF_2$, $CH_2Cl$, $CCl_3$, $CH_2F$, $-CH_2CH_2Cl$, $CHBr_2$ usw. Unter Halogen soll hier und im folgenden Fluor, Chlor, Brom oder Jod, vorzugsweise Fluor, Chlor oder Brom und insbesondere Fluor oder Chlor verstanden werden. Naphthyl steht für α- oder β-Naphthyl, vorzugsweise α-Naphthyl. Alkenyl bedeutet z. B. Propenyl-(1), Allyl, Butenyl-(1), Butenyl-(2) oder Butenyl-(3), Alkinyl steht z. B. für Propinyl-(1) oder Propargyl. Aryl bedeutet z. B. Naphthyl, insbesondere Phenyl und Aralkyl einen Niederalkylrest, der durch eine aromatische Gruppe substituiert ist, wie z. B. Benzyl oder Phenyläthyl. Cycloalkyl bedeutet je nach Zahl der Kohlenstoffatome z. B. Cyclopropyl, Cyclobutyl, Cyclopentyl, Cyclohexyl, Cycloheptyl, Cyclooctyl usw.

Die vorliegende Erfindung betrifft sowohl die freien organischen Moleküle der Formel I, als auch deren Säureadditionssalze, quaternären Azoliumsalze und Metallkomplexe. Die freien Moleküle sind bevorzugt. Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Trifluormethansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d. h. sie können ein oder mehrere organische Molekülanteile als Liganden enthalten. Komplexe mit den Metallen Kupfer, Zink, Mangan und

Zinn sind bevorzugt.

Aus der Literatur sind 1-Hydroxyäthyl-azol-Derivate als Pflanzenwachstumsregulatoren und Fungizide beispielsweise aus der europäischen Patentanmeldung 40 345 bekannt.

Die erfindungsgemässen Verbindungen der Formel I sind bei Raumtemperatur stabile Öle, Harze oder überwiegend Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel I bevorzugt. Die erfindungsgemässen Wirkstoffe der Formel I zeichnen sich durch eine sehr gute Verträglichkeit bei Kulturpflanzen aus.

Als bevorzugte Untergruppen sind diejenigen zu erwähnen, in denen die Verbindungen der Formel I dadurch gekennzeichnet sind, dass in ihnen entweder

a) Az für 1,2,4-Triazolyl steht oder
b) $R^2$ für $C_1$-$C_6$-Alkyl oder gegebenenfalls durch Halogen substituiertes Benzyl steht oder
c) $R^1$ und $R^3$ Wasserstoff oder $C_1$-$C_4$-Alkyl bedeuten oder
d) $R^5$ für durch Halogen substituiertes Phenyl steht oder
e) X Sauerstoff bedeutet.

In der Untergruppe b) sind diejenigen Verbindungen bevorzugt, in denen $R^2$ für tert.-Butyl oder i-Propyl steht.

In der Untergruppen d) sind die Verbindungen bevorzugt, in denen $R^5$ für in 4-Stellung durch Halogen substituiertes Phenyl steht.

Als besonders bevorzugte Untergruppe von Verbindungen der Formel I sind solche Verbindungen zu nennen, in denen

Az für 1,2,4-Triazolyl, $R_1$ und $R_3$ für Wasserstoff oder $C_1$-$C_4$-Alkyl,
$R_2$ für tert.-Butyl oder i-Propyl, $R_5$ für in 4-Stellung durch Halogen substituiertes Phenyl und X für Sauerstoff stehen.

Beispiele für bevorzugte Einzelverbindungen sind

1-( 4-Fluorphenoxy )-2-tert.butyl-2-hydroxy-3-fluor-3-( 1H-1,2,4-triazol-1-yl )-propan,
1-( 4-Fluorphenoxy )-2-tert.butyl-2-hydroxy-3-fluor-3-methyl-3-( 1H-1,2,4-triazol-1-yl )-propan,
1-( 4-Chlorphenoxy )-2-tert.butyl-2-hydroxy-3-fluor-3-( 1H-1,2,4-triazol-1-yl )-propan,
1-( 4-Chlorphenoxy )-2-tert.butyl-2-hydroxy-3-fluor-3-methyl-3-( 1H-1,2,4-triazol-1-yl )-propan und
1-( 4-Methylphenoxy )-2-tert.butyl-2-hydroxy-3-fluor-3-methyl-3-( 1H-1,2,4-triazol-1-yl )-propan.

Die Verbindungen der Formel I werden nach an sich bekannten Methoden hergestellt.

So erhält man die Verbindungen der Formel I durch Reaktion eines Azolylmethyloxirans der Formel II

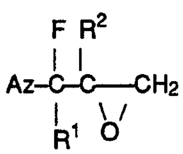

(II),

worin $R^1$, $R^2$ und Az die unter Formel I angegebene Bedeutung haben, in Gegenwart einer Base in einem inerten Lösungsmittel mit einem Alkohol oder Thioalkohol der Formel III

$$H - X - R^5 \quad \text{(III)},$$

worin X und $R^5$ die unter Formel I gegebene Bedeutung haben, umsetzt und das erhaltene Produkt der Formel Ia

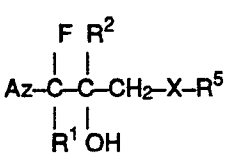

(Ia)

gewünschtenfalls durch Umsetzung mit einem Verätherungs- oder Veresterungsmittel der Formel IV

$$Y - R^3 \quad \text{(IV)},$$

worin $R^3$ die unter Formel I gegebene Bedeutung hat und Y ein Halogenatom oder einen organischen oder anorganischen Säurerest bedeutet, veräthert oder verestert.

Die Reaktion (II mit III) wird vorteilhafterweise in Gegenwart von katalytischen Mengen an Basen als Kondensationsmittel durchgeführt. Als solche kommen organische und anorganische Basen in Betracht, z. B. tertiäre Amine wie Trialkylamine (Trimethylamin, Triäthylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, LiOH, $CaH_2$, KOH, NaH $Ca(OH)_2$, $KHCO_3$, $NaHCO_3$, $CaHCO_3)_2$, $K_2CO_3$, $Na_2CO_3$), sowie Alkaliacetate wie $CH_3COONa$, oder $CH_3COOK$. Darüberhinaus eignen sich auch Alkalialkoholate wie $C_2H_5ONa$, $C_3H_7$-nONa, $(CH_3)_3C$-OK usw..

Die Reaktion (II mit III) wird bevorzugt in einem relativ polaren, jedoch reaktionsinerten organischen Lösungsmittel, durchgeführt, z. B. N,N-Dimethylformamid, N,N-Dimethylacetamid, Dimethylsulfoxid, Acetonitril, Benzonitril, Äthylenglykoldimethyläther, Diäthylenglykoldimethyläther, Triäthylenglykoldimethyläther, Dioxan, Tetrahydrofuran und anderen. Derartige Reaktionen können aber auch in Kombination mit anderen reaktionsinerten Lösungsmitteln, z. B. Benzol, Toluol, Xylol, Hexan, Petroläther, Chlorbenzol, Nitrobenzol u. a. durchgeführt werden. Die Reaktionstemperaturen liegen in einem Temperaturbereich von 20° bis 250°C, vorzugsweise 80° bis 180°C.

Die fakultative Umsetzung der Verbindungen der Unterformel Ia zu denjenigen Verbindungen der Formel I, in denen $R^3$ eine andere Bedeutung als Wasserstoff hat, wird vorteilhafterweise in einem inerten organischen Lösungsmittel durchgeführt. Geeignete Lösungsmittel sind aprotische Lösungsmittel wie N,N-Dimethylformamid, N,N-Dimethylacetamid, Dimethylsulfoxid, Acetonitril, Benzonitril, N-Methylpyrrolidinon, N-Methylpiperidinon, Benzol, Toluol, Xylol, Hexan, Cyclohexan, Chlorbenzol, Nitrobenzol und andere.

Üblicherweise steht Y für den Fall, dass die -O-$R^3$-Gruppe eine Äthergruppe ist, für Halogen wie Chlor, Brom und Jod oder für Säurereste, die sich von starken Säuren ableiten wie Schwefelsäure, Phosphorsäure, Sulfonsäuren, vorzugsweise Halogenalkylsulfonsäuren oder Halogenalkancarbonsäuren wie Trifluoressigsäure. Typische Vertreter solcher Säure-Derivate sind Dimethylsulfat, Diäthylsulfat und Trifluormethansulfonsäuremethylester. Für den Fall, dass die -O-$R^3$-Gruppe eine Estergruppe ist, steht Y im allgemeinen für Halogen wie Chlor oder Brom oder für Säurereste von Säuren, die mit dem übertragenen Acylrest ein Anhydrid bilden können. Vorzugsweise handelt es sich dabei um Anhydride mit der gleichen Säure. Dem Reagenz Y-$R^3$ entspricht dann also z. B. Essigsäureanhydrid, Propionsäureanhydrid, Benzoesäureanhydrid, Benzolsulfonsäureanhydrid oder Trifluormethansulfonsäureanhydrid.

Die Verätherung oder Veresterung der Verbindungen der Unterformel Ia geschieht mit Vorteil in Gegenwart von Basen wie Alkoholaten, Hydroxiden, Hydriden, Carbonaten oder Hydrogencarbonaten von Alkali- oder Erdalkalimetallen. Die Reaktionstemperaturen betragen 20 - 150°C, vorzugsweise 60 - 120°C.

Die Verbindungen der Formel I fallen als Diastereomerengemische an. Gegenstand der Erfindung sind alle diastereomeren Formen der Wirkstoffe der Formel I und Gemische davon. Umfasst sind somit sowohl die reinen Diastereomeren als auch die einzelnen optischen Isomeren der umfassten Enantionenpaare.

Die Alkohole oder Thioalkohole der Formel III sowie Verätherungs- und Veresterungsmittel der Formel IV sind bekannt oder werden nach an sich bekannten Methoden hergestellt.

Die Oxirane der Formel II sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel I überführen. Die Verbindungen der Formel II bildet somit einen weiteren Aspekt der vorliegenden Erfindung.

Die Oxirane der Formel II lassen sich durch Reaktion der zugrundeliegenden Ketone der Formel V

```
     F R²
     | |
Az–C–C=O          (V),
     |
     R¹
```

worin $R^1$, $R^2$ und Az die unter Formel I gegebenen Bedeutungen haben, mit Dimethylsulfoniummethylid, Dimethyloxosulfoniummethylid oder den entsprechenden Salzen wie Trimethylsulfoniumjodid oder Trimethyloxosulfoniumjodid in Gegenwart starker Basen wie Alkali- und Erdalkalialkoholate, Alkalihydroxide oder Alkali- oder Erdalkalihydride in Dimethylsulfoxid oder einem der anderen bei der Reaktion von II mit III beschriebenen Lösungsmittel herstellen. Unter geeigneten Umständen ist die Erzeugung des Sulfoniumylids bzw. die Reaktion des Sulfoniumsalzes mit der Base auch im Phasentransfer-Verfahren durchführbar. Als geeignete Phasentransfer-Katalysatoren wären verwendbar: quaternäre Ammoniumsalze wie Trialkyl-phenylalkylammoniumsalze oder Tetraalkylammoniumsalze, quaternäre Phosphoniumsalze wie Tetraalkylphosphoniumsalze oder Kronenäther wie 15-Krone-5 oder 18-Krone-6. Bei dieser Reaktion wird das Sulfoniumylid in situ erzeugt und reagiert direkt mit dem Keton der Formel V zum Oxiran der Formel II. Die Reaktion wird bei Temperaturen von 0° bis 120°C durchgeführt.

Analoge Reaktionen sind aus der Literatur bekannt; vgl. JACS, *87*, 1353 (1965). Man kann die Reaktion prinzipiell analog zu den dort beschriebenen Umsetzungen vornehmen.

Ketone der Formel V können aus den an sich bekannten α-Halogenketonen der Formel VI

```
      F R²
      | |
Hal–C–C=O          (VI)
      |
      R¹
```

worin $R^1$ und $R^2$ die unter Formel I gegebene Bedeutung haben und Hal für Chlor oder Brom steht, durch Umsetzung mit Azolen der Formel VII

H – Az (VII),

worin Az die unter Formel I gegebene Bedeutung hat, in Gegenwart einer Base hergestellt werden.

Die Ketone der Formel V können auch erhalten werden, indem man ein Azolylmethylketon der Formel VIII

$$Az\text{–}CHF\text{–}\overset{\overset{\displaystyle O}{\|}}{C}\text{–}R^2 \quad (VIII)$$

in Gegenwart einer Base mit einer Verbindung der Formel IX

$$Z - R^1 \quad (IX)$$

worin $R^1$ die unter Formel I gegebene Bedeutung hat und Z für ein Halogenatom oder einen organischen oder anorganischen Säurerest steht, umsetzt.

Die Herstellung der Ketone der Formel V erfolgt in den üblichen inerten Lösungsmitteln und gegebenenfalls bei erhöhter Temperatur.

Die Verbindungen der Formeln VI, VII, VIII, und IX sind bekannt und zum Teil im Handel erhältlich oder können nach bekannten Methoden hergestellt werden.

Die speziell für die Synthese der Verbindungen der Formel I entwickelten Zwischenprodukte der Formel V bilden einen weiteren Gegenstand der vorliegenden Erfindung.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petroläther; halogenierte Kohlenwasserstoffe wie Chlorbenzol, Methylenchlorid, Äthylenchlorid, Chloroform, Tetrachlorkohlenstoff, Tetrachloräthylen; Äther und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diäthylketon, Methyläthylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon, oder in gewissen Fällen auch Kohlendioxid.

Das beschriebene Herstellungsverfahren ist, einschliesslich aller Teilschritte, ein wichtiger Bestandteil der vorliegenden Erfindung.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies

ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z. B. mehr oder grössere Früchte zur Ausbildung kommen.

Ertragssteigerungen können in manchen Fällen auch durch einen Eingriff in den pflanzlichen Stoffwechsel, wie z. B. durch Erhöhung der Photosyntheseleistung, erreicht werden, ohne dass sich Änderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z. B. bei Tabak, Tomaten oder Kaffee, eine vollständige mechanische oder manuelle Beerntung in nur einem Arbeitsgang vorgenommen werden kann.

Durch die Anwendung von Wachtumsregulatoren kann auch die Samen- oder Knospenruhe der Pflanzen, also die endogene Jahresrhythmik, beeinflusst werden, so dass die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z. B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbedeckungspflanzen, den sogenannten Cover crops. In tropischen und subtropischen Monokulturen, wie z. B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogegen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder

vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z. B. Venturia, Podosphära, Erysiphe, Monilinia, Uncinula); Basidiomyceten (z. B. die Gattungen Hemileia, Rhizoctonia, Puccinia); Fungi imperfecti (z. B. Botrytis, Helminthosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung über auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 2 kg AS/ha, insbesondere bei 200 g bis 600 g AS/ha.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl oder -äthyläther, Ketone wie Cyclohexanon, stark po-

lare Lösungsmittel wie N-Methyl-2-pyrrolidon, Dimethylsulfoxid oder Dimethylformamid, sowie gegebenenfalls epoxidiertes Pflanzenöl wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkli-, Erdalkali- oder gegebenenfalls substituierte Ammoniumsalze von höheren Fettsäuren ($C_{10}$ - $C_{22}$), wie z. B. die Na- oder K-Salze der Öl oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykolätherguppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben:

"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I - III, Chemical Publishing Co., New York, 1980 - 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 %, eines Tensides.

Insbesondere setzen sich bevorzugte Formulierungen folgendermassen zusammen: (% = Gewichtsprozent)

**Lösungen**

| | |
|---|---|
| Aktiver Wirkstoff: | 5 bis 95 %, vorzugsweise 10 bis 80 % |
| Lösungsmittel: | 95 bis 5 %, vorzugsweise 90 bis 20 % |
| oberflächenaktives Mittel: | 1 bis 30 %, vorzugsweise 2 bis 20 %. |

**Emulgierbare Konzentrate**

| | |
|---|---|
| Aktiver Wirkstoff | 10 bis 50 %, bevorzugt 10 bis 40 % |
| oberflächenaktives Mittel: | 5 bis 30 %, vorzugsweise 10 bis 20 % |
| flüssiges Trägermittel: | 20 bis 95 %, vorzugsweise 40 bis 80 %. |

**Stäube**

| | |
|---|---|
| Aktiver Wirkstoff: | 0,5 bis 10 %, vorzugsweise 2 bis 8 % |
| festes Trägermaterial | 99,5 bis 90 %, vorzugsweise 98 bis 92 %. |

**Suspensions-Konzentrate**

| | |
|---|---|
| Aktiver Wirkstoff | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

**Benetzbare Pulver**

| | |
|---|---|
| Aktiver Wirkstoff: | 5 bis 90 %, vorzugsweise 10 bis 80 % und insbesondere 20 bis 60 % |
| oberflächenaktives Mittel: | 0,5 bis 20 %, vorzugsweise 1 bis 15 % |
| festes Trägermaterial: | 5 bis 90 %, vorzugsweise 30 bis 70 %. |

**Granulate:**

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben.

**Herstellungsbeispiele**

**Beispiel 1:**

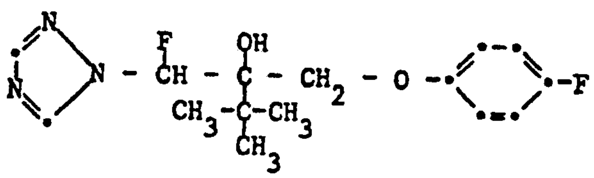

**1-(4-Fluorphenoxy)-2-tert.butyl-2-hydroxy-3-fluor-3-(1H-1,2,4-triazol-1-yl)propan (Verbindung 5.6)**

a) 1-Fluor-1-(1H-1,2,4-triazol-1-yl)-3,3-dimethyl-2-butanon:

Zu einer Mischung von 23,0 g 1,2,4-Triazol und 44,2 g Kaliumcarbonat in 280 ml Äthylmethylketon werden langsam 63,0 g 1-Brom-1-fluor-3,3-dimethyl-2-butanon zugesetzt. Das Reaktionsgemisch wird für 18 Stunden bei 45 - 50°C gerührt, anschliessend filtriert und eingedampft. Der Rückstand wird in Methylenchlorid aufgenommen und mit Wasser gewaschen. Nach dem Trocknen und Eindampfen der organischen Phase erhält man 50,0 g 1-Fluor-1-(1H-1,2,4-triazol-1-yl)-3,3-dimethyl-2-butanon, Smp. 54 - 55°C.

b) 2-tert.Butyl-2-[(1H-1,2,4-triazol-1-yl)-fluormethyl]-oxiran:

Eine Mischung von 20 g 1-Fluor-1-(1H-1,2,4-triazol-1-yl)-3,3-dimethyl-2-butanon, 13,5 g Kaliumtertiärbutylat, 24,0 g Trimethylsulfoxoniumiodid, 80 ml Tetrahydrofuran und 20 ml Dimethylsulfoxid wird für 6 Stunden bei 60°C gerührt. Anschliessend wird das Reaktionsgemisch mit Eiswasser aufgenommen und mit Äthylacetat extrahiert. Die vereinigten organischen Phasen werden mit Wasser und gesättigter Kochsalzlösung gewaschen, über Natriumsulfat getrocknet und eingedampft.

Durch Kristallisation aus Petroläther erhält man 6,9 g 2-tert.Butyl-2-[(1H-1,2,4-triazol-1-yl)-fluormethyl]-oxiran, Smp. 72 - 74°.

c) 3,0 g 2-tert.Butyl-2-[(1H-1,2,4-triazol-1-yl)-fluormethyl]-oxiran, 1,7 g 4-Fluorphenol und 0,3 g Kalium-p-fluorphenolat werden in 10 ml Diäthylenglykoldimethyläther für 5 Stunden bei 140°C gerührt. Nach dem Abkühlen wird die Mischung mit Eiswasser aufgenommen und mit Äthylacetat extrahiert. Die vereinigten organischen Phasen werden mit Wasser, gesättigter Natriumchloridlösung, 2N Natriumhydroxidlösung und wieder gesättigter Natriumchloridlösung gewaschen, über Natriumsulfat getrocknet und eingedampft. Durch Kristallisation aus Äther/HexanGemisch (1 : 4) erhält man 2,3 g 1-(4-Fluorphenoxy)-2-tert.butyl-2-hydroxy-3-fluor-3-(1H-1,2,4-triazol-1-yl)-propan, Smp. 89 - 90°C

**Beispiel 2:**

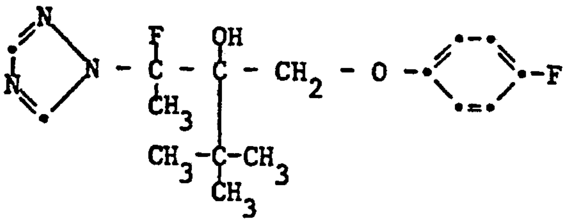

**1-(4-Fluorphenoxy)-2-tert.butyl-2-hydroxy-3-fluor-3-methyl-3-(1H-1,2,4-triazol-1-yl)-propan (Verbindung 5.7).**

a) 2-Fluor-2-(1H-1,2,4-triazol-1-yl)-4,4-dimethyl-3-pentanon:
Zu einer Lösung von 13,5 g Kaliumtertiärbutylat in 100 ml Tetrahydrofuran lässt man 20,0 g 1-Fluor-1-(1H-1,2,4-triazol-1-yl)-3,3-dimethyl-2-butanon zutropfen. Nachdem das Gemisch für 0,5 Stunden gerührt worden ist lässt man 15,6 g Methyljodid zutropfen und rührt für 18 Stunden bei 60°C. Nach dem Abkühlen wird die Reaktiosmischung mit Eiswasser aufgenommen und mit Äthylacetat extrahiert. Die vereinigten organischen Phasen werden mit Wasser und gesättigter Kochsalzlösung gewaschen, über Natriumsulfat getrocknet und eingedampft.

Durch Destillation des öligen Rückstandes erhält man 14,0 g 2-Fluor-2-(1H-1,2,4-triazol-1-yl)-4,4-dimethyl-3-pentanon, Sdp 48 - 50°C/0,013 mbar,

b) 2-tert.Butyl-2-[1-fluor-1-(1H-1,2,4-triazol-1-yl)-äthyl]oxiran:
Zu einer Mischung von 13,2 g Trimethylsulfoxoniumjodid, 7,3 g Kaliumtertiärbutylat, 20 ml Dimethylsulfoxid und 80 ml Tetrahydrofuran lässt man 12,0 g 2-Fluor-2-(1H-1,2,4-triazol-1-yl)-4,4-dimethyl-3-pentanon zutropfen und rührt das Reaktionsgemisch für 18 Stunden bei 80°C. Nach dem Abkühlen wird das Gemisch in Eiswasser aufgenommen und mit Äthylacetat extrahiert. Die vereinigten organischen Phasen werden mit Wasser und gesättigter Kochsalzlösung gewaschen, über Natriumsulfat getrocknet und eingedampft. Durch Destillation des öligen Rückstandes erhält man 6 g 2-tert.Butyl-2-[1-fluor-1-(1H-1,2,4-triazol-1-yl)-äthyl]-oxiran, Sdp. 60 - 61°C/0,013 mbar.

c) 6,0 g 2-tert.Butyl-2-[1-fluor-1-(1H-1,2,4-triazol-1-yl)-äthyl]-oxiran, 3,1 g 4-Fluorphenol und 0,4 g Kalium-p-fluorphenolat werden für 6 Stunden in 20 ml Diäthylenglykoldimethyläther auf 140°C erhitzt. Nach dem Abkühlen wird die Reaktionsmischung im Eiswasser aufgenommen und mit Äthylacetat extrahiert. Die vereinigten organischen Phasen werden nacheinander mit Wasser, gesättigter Kochsalzlösung, 2N Natriumhydroxyidlösung, Wasser und gesättigter Kochsalzlösung gewaschen, über Natriumsulfat getrocknet und eingedampft. Durch Chromatographie an Kieselgel mit Hexan/Äthylacetat-Gemisch (4 : 1) erhält man 1,6 g 1-(4-Fluorphenoxy)-2-tert.butyl-2-hydroxy-3-fluor-3-methyl-3-(1H-1,2,4-triazol1-yl)-propan als Diastereomerengemisch,
Smp. 90 - 93°C.

In analoger Weise werden die in den folgenden Tabellen aufgeführten Verbindungen hergestellt.

**Tabelle 1:**

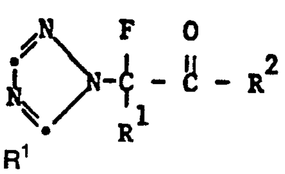

| Nr. | $R^1$ | $R^2$ | physikalische Daten |
|---|---|---|---|
| 1.1 | H | $C_4H_9$–t | Smp. 54 – 55°C |
| 1.2 | H | $C_3H_7$–i | |
| 1.3 | $CH_3$ | $C_4H_9$–t | Sdp. 48 – 50°C/0,013 mbar |
| 1.4 | $CH_3$ | $C_3H_7$–i | |
| 1.5 | H | 4–Cl–$C_6H_4$–$CH_2$– | |
| 1.6 | H | 4–F–$C_6H_4$–$CH_2$– | |

**Tabelle 2:**

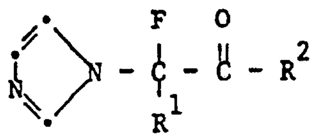

| Nr. | $R^1$ | $R^2$ | physikalische Daten |
|---|---|---|---|
| 2.1 | H | $C_4H_9$–t | |
| 2.2 | H | $C_3H_7$–i | |
| 2.3 | $CH_3$ | $C_4H_9$–t | |
| 2.4 | $CH_3$ | $C_3H_7$–i | |
| 2.5 | H | 4–Cl–$C_6H_4$–$CH_2$– | |
| 2.6 | $CH_3$ | 4–Cl–$C_6H_4$–$CH_2$– | |

**Tabelle 3:**

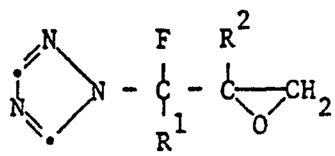

| Nr. | $R^1$ | $R^2$ | physikalische Daten |
|---|---|---|---|
| 3.1 | H | $C_4H_9$–t | Smp. 72 – 74°C |
| 3.2 | H | $C_3H_7$–i | |
| 3.3 | $CH_3$ | $C_4H_9$–t | Sdp. 60 – 61°C/0,013 mbar |
| 3.4 | $CH_3$ | $C_3H_7$–i | |
| 3.5 | H | 4–Cl–$C_6H_4$–$CH_2$– | |
| 3.6 | H | 4–F–$C_6H_4$–$CH_2$– | |
| 3.7 | $C_2H_5$ | $C_4H_9$–t | |
| 3.8 | $C_6H_5$–$CH_2$– | $C_4H_9$–t | |
| 3.9 | 4–Cl–$C_6H_4$–$CH_2$– | $C_4H_9$–t | |
| 3.10 | 4–F–$C_6H_4$–$CH_2$ | $C_4H_9$–t | |
| 3.11 | $C_3H_7$–n | $C_4H_9$–t | |

**Tabelle 4:**

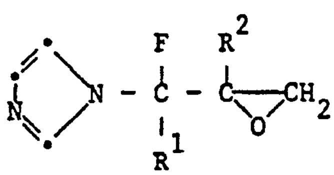

| Nr. | $R^1$ | $R^2$ | physikalische Daten |
|---|---|---|---|
| 4.1 | H | $C_4H_9$–t | |
| 4.2 | H | $C_3H_7$–i | |
| 4.3 | $CH_3$ | $C_4H_9$–t | |
| 4.4 | $CH_3$ | $C_3H_7$–i | |
| 4.5 | H | 4–Cl–$C_6H_4$–$CH_2$– | |
| 4.6 | $CH_3$ | 4–Cl–$C_6H_4$–$CH_2$– | |
| 4.7 | $C_2H_5$ | $C_4H_9$–t | |

**Tabelle 5:**

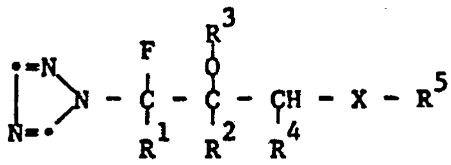

| Nr. | $R^1$ | $R^2$ | $R^3$ | $R^4$ | $R^5$ | X | physikalische Daten |
|---|---|---|---|---|---|---|---|
| 5.1 | H | $C_4H_9$–t | H | H | 4–Cl–$C_6H_4$– | O | Smp. 100 – 101°C |
| 5.2 | $CH_3$ | $C_4H_9$–t | H | H | 4–Cl–$C_6H_4$– | O | Smp. 98 – 99°C |
| 5.3 | $C_2H_5$ | $C_4H_9$–t | H | H | 4–Cl–$C_6H_4$– | O | |
| 5.4 | 4–Cl–$C_6H_4$–$CH_2$– | $C_4H_9$–t | H | H | 4–Cl–$C_6H_4$– | O | |
| 5.5 | 4–F–$C_6H_4$–$CH_2$– | $C_4H_9$–t | H | H | 4–Cl–$C_6H_4$– | O | |
| 5.6 | H | $C_4H_9$–t | H | H | 4–F–$C_6H_4$– | O | Smp. 89 – 90°C |
| 5.7 | $CH_3$ | $C_4H_9$–t | H | H | 4–F–$C_6H_4$– | O | Smp. 90 – 93°C |
| 5.8 | $C_2H_5$ | $C_4H_9$–t | H | H | 4–F–$C_6H_4$– | O | |
| 5.9 | 4–Cl–$C_6H_4$–$CH_2$– | $C_4H_9$–t | H | H | 4–F–$C_6H_4$– | O | |
| 5.10 | 4–F–$C_6H_4$–$CH_2$– | $C_4H_9$–t | H | H | 4–F–$C_6H_4$– | O | |
| 5.11 | H | $C_4H_9$–t | $CH_3$ | H | 4–F–$C_6H_4$– | O | |
| 5.12 | H | $C_4H_9$–t | $C_6H_5$–$CH_2$– | H | 4–F–$C_6H_4$– | O | |
| 5.13 | H | $C_4H_9$–t | H | H | 4–Br–$C_6H_4$– | O | Smp. 108 – 109°C |
| 5.14 | H | $C_4H_9$–t | H | H | 4–$CH_3$–$C_6H_4$– | O | Smp. 96 –98°C |
| 5.15 | H | $C_4H_9$–t | H | H | $C_6H_5$ | O | |
| 5.16 | H | $C_4H_9$–t | H | H | 2–Cl—Cl–$C_6H_3$– | O | Smp. 127 – 128°C |
| 5.17 | H | $C_4H_9$–t | H | H | 4–F–$C_6H_4$– | S | |
| 5.18 | $CH_3$ | $C_4H_9$–t | H | H | 4–Br–$C_6H_4$– | O | |
| 5.19 | $C_2H_5$ | $C_4H_9$–t | H | H | 4–Br–$C_6H_4$– | O | |
| 5.20 | $CH_3$ | $C_4H_9$–t | H | H | 4–$CH_3$–$C_6H_4$– | O | Smp.136 – 137°C |
| 5.21 | $CH_3$ | $C_4H_9$–t | H | H | $C_6H_5$ | O | |
| 5.22 | $CH_3$ | $C_4H_9$–t | $CH_3$ | H | 4–F–$C_6$–$H_4$– | O | |
| 5.23 | $CH_3$ | $C_4H_9$–t | $CH_3$ | H | 4–Cl–$C_6H_4$ | O | |
| 5.24 | $CH_3$ | $C_4H_9$–t | $C_6H_5$–$CH_2$– | H | 4–F–$C_6H_4$– | O | |
| 5.25 | $CH_3$ | $C_4H_9$–t | 4–Cl–$C_6H_4$–$CH_2$– | H | 4–F–$C_6H_4$– | O | |
| 5.26 | $CH_3$ | $C_4H_9$–t | $C_6H_5$–$CH_2$ | H | 4–Cl–$C_6H_4$– | O | |
| 5.27 | $CH_3$ | $C_4H_9$–t | 4–Cl–$C_6H_4$–$CH_2$– | H | 4–F–$C_6H_4$– | O | |
| 5.28 | $C_2H_5$ | $C_4H_9$–t | $CH_3$ | H | 4–F–$C_6H_4$– | O | |
| 5.29 | $CH_3$ | $C_4H_9$–t | –$COCH_3$ | H | 4–F–$C_6H_4$– | O | |
| 5.30 | $CH_3$ | $C_4H_9$–t | –$COCH_3$ | H | 4–Cl–$C_6H_4$– | O | |
| 5.31 | H | $C_4H_9$–t | H | H | 2–Cl–$C_6H_4$– | O | Smp. 121 – 122°C |
| 5.32 | H | $C_4H_9$–t | H | H | 2–Cl–C1–$C_6H_5$– | O | Smp. 163 – 165°C |
| 5.33 | H | $C_4H_9$–t | H | H | 2–Cl—Cl–$C_6H_5$– | O | Smp. 135 – 136°C |
| 5.34 | H | $C_4H_9$–t | H | H | $C_4H_9$–n | S | Smp. 150 – 160°C/ 0,06 mb |
| 5.35 | $CH_3$ | $C_4H_9$–t | H | H | 2–$CH_3$—$CH_3$–$C_6H_3$– | O | Smp. 147 – 148°C |
| 5.36 | $CH_3$ | $C_4H_9$–t | H | H | 2–Cl–$C_6H_4$– | O | Smp. 158 – 159°C |
| 5.37 | $CH_3$ | $C_4H_9$–t | H | H | $C_4H_9$–n | S | Smp. 125°C/ 0,01 mb |

Tabelle 6:

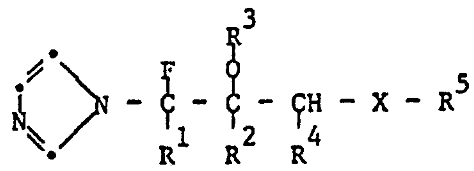

| Nr. | $R^1$ | $R^2$ | $R^3$ | $R^4$ | $R^5$ | X | physikalische Daten |
|---|---|---|---|---|---|---|---|
| 6.1 | H | $C_4H_9$–t | H | H | 4–Cl–$C_6H_4$– | O | |
| 6.2 | H | $C_4H_9$–t | H | H | 4–F–$C_6H_4$– | O | |
| 6.3 | H | $C_4H_9$–t | H | H | 4–$CH_3$–$C_6H_4$– | O | |
| 6.4 | H | $C_4H_9$–t | H | H | 4–Br–$C_6H_4$– | O | |
| 6.5 | H | $C_4H_9$–t | H | H | 4–$OCF_3$–$C_6H_4$– | O | |
| 6.6 | H | $C_4H_9$–t | H | H | 2–Cl–4–Cl–$C_6H_4$– | O | |
| 6.7 | $CH_3$ | $C_4H_9$–t | H | H | 4–Cl–$C_6H_4$– | O | |
| 6.8 | $CH_3$ | $C_4H_9$–t | H | H | 4–F–$C_6H_4$– | O | |
| 6.9 | $C_2H_5$ | $C_4H_9$–t | H | H | 4–F–$C_6H_4$– | O | |

**Formulierungsbeispiele**

**Beispiel 3:**

Formulierungsbeispiele für flüssige Wirkstoffe der Formel I (% = Gewichtsprozent)

| a) **Emulsionskonzentrate** | a) | b) | c) |
|---|---|---|---|
| Wirkstoff | 20 % | 40 % | 50 % |
| Ca–Dodecylbenzolsulfonat | 5 % | 8 % | 5,8 % |
| Ricinusöl–polyäthylenglykoläther (36 Mol AeO) | 5 % | – | – |
| Tributylphenol–polyäthylenglykoläther (30 Mol AeO) | – | 12 % | 4,2 % |
| Cyclohexanon | – | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) **Lösungen** | a) | b) | c) | d) |
|---|---|---|---|---|
| Wirkstoff | 80 % | 10 % | 5 % | 95 % |
| Äthylenglykol–monomethyl–äther | 20 % | – | – | – |
| Polyäthylenglykol MG 400 | – | 70 % | – | – |
| N–Methyl–2–pyrrolidon | – | 20 % | – | – |
| Epoxidiertes Kokosnussöl | – | – | 1 % | 5 % |
| Benzin (Siedegrenzen 160 – 190°C) | – | – | 94 % | – |

Die Lösungen sind zur Anwendung in Form kleinster Tropfen geeignet.

| c) **Granulate** | a) | b) |
|---|---|---|
| Wirkstoff | 5 % | 10 % |
| Kaolin | 94 % | – |
| Hochdisperse Kieselsäure | 1 % | – |
| Attapulgit | – | 90 % |

Der Wirkstoff wird in Methylenchlorid gelöst, auf den Träger aufgesprüht und das Lösungsmittel anschliessend im

Vakuum abgedampft.

d) **Stäubemittel**

| | a) | b) |
|---|---|---|
| Wirkstoff | 2 % | 5 % |
| Hochdisperse Kieselsäure | 1 % | 5 % |
| Talkum | 97 % | – |
| Kaolin | – | 90 % |

Durch inniges Vermischen der Trägerstoffe mit dem Wirkstoff erhält man gebrauchsfertige Stäubemittel.

**Beispiel 4:**

Formulierungsbeispiele für feste Wirkstoffe der Formel I (% = Gewichtsprozent)

a) **Spritzpulver**

| | a) | b) |
|---|---|---|
| Wirkstoff | 20 % | 60 % |
| Na–Ligninsulfonat | 5 % | 5 % |
| Na–Laurylsulfat | 3 % | – |
| Na–Diisobutylnaphthalinsulfonat | – | 6 % |
| Octylphenolpolyäthylenglykoläther (7 – 8 Mol AeO) | – | 2 % |
| Hochdisperse Kieselsäure | 5 % | 27 % |
| Kaolin | 67 % | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) **Emulsions-Konzentrat**

| | |
|---|---|
| Wirkstoff | 10 % |
| Octylphenolpolyäthylenglykoläther (4 – 5 Mol AeO) | 3 % |
| Ca–Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) **Stäubemittel**

| | a) | b) |
|---|---|---|
| Wirkstoff | 5 % | 8 % |
| Talkum | 95 % | – |
| Kaolin | – | 92 % |

Man erhält anwendungsfertige Stäubemittel, indem der Wirkstoff mit dem Träger vermischt und auf einer geeigneten Mühle vermahlen wird.

d) **Extruder Granulat**

| | |
|---|---|
| Wirkstoff | 10 % |
| Na–Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) **Umhüllungs-Granulat**

| | |
|---|---|
| Wirkstoff | 3 % |

| | |
|---|---|
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) **Suspensions-Konzentrat**

| | |
|---|---|
| Wirkstoff | 40% |
| Äthylenglykol | 10 % |
| Nonylphenolpolyäthylenglykoläther (15 Mol AeO) | 6 % |
| Na–Ligninsulfonat | 10 % |
| Carboxymethylcellulose | 1 % |
| 37 %–ige wässrige Formaldehyd–Lösung | 0,2 % |
| Silikonöl in Form einer 75 %–igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele**

**Beispiel 5: Wirkung gegen Puccinia graminis auf Weizen**

a) **Residual-protektive Wirkung**

Weizenpflanzen werden 6 Tage nach der Aussaat mit einer aus Spritzpulver des Wirkstoffes hergestellten Spritzbrühe (0,06 % Aktivsubstanz) besprüht. Nach 24 Stunden werden die behandelten Pflanzen mit einer Uredosporensuspension des Pilzes infiziert. Nach einer Inkubation während 48 Stunden bei 95 - 100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) **Systemische Wirkung**

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95 - 100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus der Tabelle 1 zeigen gegen Puccinia-Pilze eine gute Wirkung. Unbehandelte aber infizierte Kontrollpflanzen zeigen einen Puccinia-Befall von 100 %. Unter anderem hemmen die Verbindungen 5.6 und 5.7 den Puccinia-Befall auf 0 bis 5 %.

**Beispiel 6: Wirkung gegen Cercospora arachidicola auf Erdnusspflanzen**

a) **Residual-protektive Wirkung**

Zu 10 - 15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) **Systemische Wirkung**

Zu 10 - 15 cm hohen Erdnusspflanzen wird eine aus Spritzpulver des Wirkstoffes hergestellte Spritzbrühe gegossen (0,06 % Aktivsubstanz bezogen auf das Erdvolumen). Nach 48 Stunden werden die behandelten Pflanzen mit einer Konidiensuspension des Pilzes infiziert und 72 Stunden bei ca. 21°C und hoher Luftfeuchtigkeit inkubiert. Anschliessend werden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100 %), zeigen Erdnusspflanzen, die mit Wirkstoffen aus der Tabelle 1 behandelt werden, einen stark reduzierten Cercospora-Befall. So verhindern die Verbindungen 5.6 und 5.7 in obigen Versuchen das Auftreten von Flecken fast vollständig (0 - 10 %).

**Beispiel 7: Wirkung gegen Erysiphe graminis auf Gerste**

a) **Residual-protektive Wirkung**

Ca. 8 cm hohe Gerstenpflanzen werden mit einer aus Spritzpulver des Wirkstoffes hergestellten Spritzbrühe (0,02 % Aktivsubstanz) besprüht. Nach 3 - 4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) **Systemische Wirkung**

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen der Formel I zeigen eine gute Wirkung gegen Erysiphe-Pilze. Unbehandelte, aber infizierte Kontrollpflanzen zeigen einen Erysiphe-Befall von 100 %. Unter anderen Verbindungen aus der Tabelle 1 hemmen die Verbindungen Nr. 5.6 und 5.7 den Pilzbefall bei Gerste auf 0 bis 5 %.

**Beispiel 8: Residual-protektive Wirkung gegen Venturia inaequalis auf Apfeltrieben**

Apfelstecklinge mit 10 - 20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relativer Luftfeuchtigkeit inkubiert und während 10 weiteren Tagen in einem Gewächshaus bei 20 - 24°C aufgestellt. Der Schorfbefall wird 15 Tage nach der Infektion beurteilt. Verbindungen 5.6, 5.7 und andere hemmen den Krankheitsbefall auf weniger als 10 %. Unbehandelte aber infizierte Triebe zeigen einen 100 %-igen Venturia-Befall.

**Beispiel 9: Wirkung gegen Botrytis cinerea auf Bohnen**

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95 - 100 % relativer Luftfeuchtigkeit und 21°C erfolgt die Beurteilung des Pilzbefalles. Die Verbindungen aus der Tabelle 1 hemmen in vielen Fällen die Pilzinfektion sehr stark. Bei einer Konzentration von 0,02 % erweisen sich z. B. die Verbindungen Nr. 5.6, 5.7 und 5.14 als voll wirksam (Krankheitsbefall 0 bis 5 %). Der Botrytis-Befall unbehandelter aber infizierter Bohnenpflanzen betrug 100 %.

**Beispiel 10: Wuchshemmung bei Getreide**

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt umgerechnet 0.3, 1 bzw. 3 kg Aktivsubstanz pro Hektar. 21 Tage nach Applikation wird das Wachstum des Getreides beurteilt. Hierbei kann festgestellt werden, dass Getreidepflanzen, die mit Wirkstoffen der Formel I behandelt worden sind, im Vergleich zu unbehandelten Kontrollpflanzen eine starke Wuchsreduktion aufweisen.

**Testresultate:** Wuchshöhe der Getreidepflanzen in Prozent zur Wuchshöhe von unbehandelten Kontrollpflanzen.

| Verb.Nr. | 3kgAS/ha | | 1 kgAS/ha | | 0,3 kgAS/ha | |
|---|---|---|---|---|---|---|
| | Roggen | Gerste | Roggen | Gerste | Roggen | Gerste |
| 5.1 | 28 | 44 | 36 | 53 | 50 | 67 |
| 5.6 | 5 | 6 | 23 | 21 | 38 | 53 |
| 5.7 | 12 | 19 | 18 | 37 | 46 | 65 |
| 5.14 | 43 | 67 | 57 | 84 | 80 | 86 |
| 5.20 | 28 | 67 | 38 | 86 | 58 | 88 |

**Beispiel 11: Wuchshemmung bei Gräsern**

In Kunststoffschalen mit Erde-Torf-Sand-Gemisch (6 : 3 : 1) wird ein Grasgemisch enthaltend Poa pratensis, Dactylis glomerata, Lolium perenne, Festuca rubra, Festuca ovina, Cynosurus crystatus, Agropyron repens und Bromus inermis im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirksstoffes der Formel I besprüht. Die Wirkstoffmenge beträgt umgerechnet 0.3, 1 bzw. 3 kg Aktivsubstanz pro Hektar. 21 Tage nach der Applikation wird das Durchschnitts-Wachstum der Gräser beurteilt, dabei zeigt es sich, dass die erfindungsgemässen Wirkstoffe aus den Tabellen 5 und 6 eine merkliche Wuchshemmung bewirken.

**Testresulate:** Wuchshöhe von Gräsern in Prozent zur Wuchshöhe der unbehandelten Kontrollpflanzen.

| Verb. Nr. | 3 kgAS/ha | 1 kgAS/ha | 0,3 kgAS/ha |
|---|---|---|---|
| 5.1 | 39 | 39 | 61 |
| 5.6 | 31 | 33 | 39 |
| 5.7 | 32 | 36 | 50 |
| 5.14 | 76 | 83 | 86 |
| 5.20 | 64 | 79 | 89 |

**Beispiel 12: Ertragssteigerung an Sojabohnen**

In Kunstoffbehältern mit einem Erde-Torf-Sandgemisch im Verhältnis 6 : 3 : 1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5 - 6 Trifola-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt umgerechnet bis zu 3 kg Aktivsubstanz pro Hektar. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der geernteten Schoten.

**Beispiel 13: Hemmung des Vegetativ-Wachstums an Soja**

In Kunststofftöpfen mit einem Erde-Torf-Sandgemisch im Verhältnis 6 : 3 : 1 werden die Sojabohnen der Sorte "Hark" angesät, im Gewächshaus aufgestellt und nach Bedarf bewässert. 15 Tage nach der Saat werden die Pflanzen mit der wässrigen Spritzbrühe eines Wirkstoffes der Formel I bis zur Benetzung besprüht. Die Wirkstoffkombination beträgt umgerechnet 0.1, 0.5 bzw. 1.5 kg Aktivsubstanz pro Hektar. 14 Tage nach der Applikation wird das Wachstum der Pflanzen beurteilt. Dabei zeigt sich dass die erfindungsgemässen Wirkstoffe aus den Tabellen 5 und 6 eine merkliche Wuchshemmung bewirken.

**Testresultate:** Wuchshöhe von Sojapflanzen in Prozent zur Wuchshöhe von unbehandelten Kontrollpflanzen

| Verb. Nr. | 1,5 kgAS/ha | 0,5 kgAS/ha | 0,1 kgAS/ha |
|---|---|---|---|
| 5.1 | 11 | 11 | 11 |
| 5.6 | 5 | 5 | 11 |
| 5.7 | 5 | 5 | 11 |
| 5.14 | 19 | 19 | 48 |
| 5.20 | 16 | 16 | 16 |

**Beispiel 14: Wuchshemmung bei Bodenbedecker-Pflanzen (Cover Crops)**

In Kunststoffschalen mit Erde-Torf-Sand-Gemisch (1 : 1 : 1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 5 Tage nach dem Zurückschneiden mit einer Spritzbrühe des Wirkstoffes (umgerechnet 0.1, 0.3, 1 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 5 und 6 eine deutliche Wuchshemmung der Bodenbedecker-Pflanzen auslösen.

**Testresultate:** Neuzuwachs von cover-crop-Pflanzen in Prozent an Frischgewicht und Wuchshöhe zum Neuzuwachs der unbehandelten Kontrollpflanzen

| Verb. Nr. | kgAS/ha | Neuzuwachs von centrosema Frischgewicht | pubescens Wuchshöhe | Psophocarpus Frischgewicht | palustris Wuchshöhe |
|---|---|---|---|---|---|
| 5.1 | 3 | 15 | 10 | 29 | 10 |
| | 1 | 26 | 10 | 35 | 10 |
| | 0,3 | 26 | 10 | 39 | 40 |
| | 0,1 | 59 | 30 | 59 | 30 |
| 5.6 | 3 | 15 | 10 | 26 | 10 |
| | 1 | 14 | 10 | 35 | 10 |
| | 0,3 | 30 | 10 | 23 | 10 |
| | 0,1 | 41 | 10 | 58 | 40 |
| 5.20 | 3 | 63 | 30 | 29 | 10 |
| | 1 | 59 | 40 | 58 | 40 |
| | 0,3 | 81 | 80 | 84 | 80 |
| | 0,1 | 85 | 80 | 97 | 100 |

**Beispiel 15: Wuchsterminierung Baumwolle**

In Kunststoffbehältern mit einem- Erde-Torf-Gemisch im Verhältnis von 2 : 1 werden Baumwollpflanzen der Sorte Delta Pine angesät und im Gewächshaus bei Temperaturen von 20° - 26°C angezogen. Nach 2 Monaten haben sich die Pflanzen bis zum 6 Blattstadium entwickelt. Zu diesem Zeitpunkt werden die Pflanzen mit einer wässrigen Dispersion eines Wirkstoffes der Formel I bis zur guten Benetzung besprüht. Die Wirkstoffkonzentration beträgt umgerechnet 2,0 kg Aktivsubstanz pro Hektar. Die Auswertung erfolgt ca. 1 Monat nach der Applikation des Wirkstoffs. Im Vergleich zu unbehandelten Kontrollpflanzen bewirken die erfindungsgemässen Wirkstoffe der Formel I eine merkliche Reduktion des Neuzuwachses.

**Patentansprüche** für die Vertragsstaaten: BE, CH, LI, DE, FR, IT, NL, SE, GB

1. Fluorazolylpropan-Derivate der Formel I

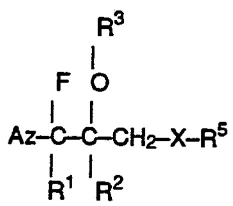

(I)

worin
Az für Imidazolyl oder 1,2,4-Triazolyl steht,
$R^1$ für Wasserstoff, für gegebenenfalls durch Halogen oder Cyan substituiertes $C_1$-$C_{68}$-Alkyl oder für gegebenenfalls durch Halogen, $c_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl steht,
$R^2$ gegebenenfalls durch $C_1$-$C_3$-Alkoxy, Phenyl oder $C_1$-$C_3$-Phenylalkyl substituiertes $C_1$-$C_{10}$-Alkyl bedeutet, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,
$R^3$ für Wasserstoff, $C_1$-$C_6$-Alkyl, $C_1$-$C_6$-Alkylcarbonyl oder gegebenenfalls durch Halogen, $C_1$-$C_3$-Alkoxy, $C_1$-$C_3$-Alkyl, $C_1$-$C_3$-Halogenalkyl, Cyan, Carboxyl oder $C_1$-$C_6$-Alkoxycarbonyl substituiertes $C_1$-$C_3$-Phenylalkyl steht,
$R^5$ für einen unsubstituierten oder ein- oder mehrfach substituierten Rest steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl, wobei die Substituenten aus der Reihe Halogen, Cyano, $C_1$-$C_3$-Alkyl, $C_1$-$C_5$-Alkoxy, $C_1$-$C_5$-Haloalkyl, $C_1$-$C_3$-Alkylthio, $C_1$-$C_3$-Haloalkylthio, $C_1$-$C_3$-Haloalkyl, Nitro und/oder Rhodano ausgewählt sind; und
X Sauerstoff oder Schwefel bedeutet; unter Einschluss der Säureadditionssalze quaternären Azoliumsalze und Metallkomplexe.

2. Verbindungen gemäss Anspruch 1, dadurch gekennzeichnet, dass Az für 1,2,4-Triazolyl steht.

3. Verbindungen gemäss Anspruch 1, dadurch gekennzeichnet, dass $R^2$ $C_1$-$C_6$-Alkyl oder gegebenenfalls durch Halogen substituiertes Benzyl bedeutet.

4. Verbindungen gemäss Anspruch 3, dadurch gekennzeichnet, dass $R^2$ für tert.-Butyl oder i-Propyl steht.

5. Verbindungen gemäss Anspruch 1, dadurch gekennzeichnet, dass $R^1$ und $R^3$ Wasserstoff oder $C_1$-$C_4$ Alkyl bedeuten.

6. Verbindungen gemäss Anspruch 1, dadurch gekennzeichnet, dass $R^5$ für durch Halogen substituiertes Phenyl steht.

7. Verbindungen gemäss Anspruch 1, dadurch gekennzeichnet, dass $R^5$ für in 4-Stellung durch Halogen substituiertes Phenyl steht.

8. Verbindungen gemäss Anspruch 1, dadurch gekennzeichnet, dass X Sauerstoff ist.

9. Verbindungen gemäss Anspruch 1, dadurch gekennzeichnet, dass Az für 1,2,4-Triazolyl, $R^1$ und $R^3$ für Wasserstoff oder $C_1$-$C_4$-Alkyl, $R^2$ für tert.-Butyl oder i-Propyl, $R^5$ für in 4-Stellung durch Halogen substituiertes Phenyl und X für Sauerstoff stehen.

10. 1-(4-Fluorphenoxy)-2-tert.butyl-2-hydroxy-3-fluor-3-(1H-1,2,4-triazol-1-yl)propan gemäss Anspruch 1.

11. 1-(4-Fluorphenoxy)-2-tert.butyl-2-hydroxy-3-fluor-3-methyl-3-(1H-1,2,4-triazol-1-yl)-propan gemäss Anspruch 1.

12. 1-(4-Chlorphenoxy)-2-tert.butyl-2-hydroxy-3-fluor-3-(1H-1,2,4-triazol-1-yl)-propan gemäss Anspruch 1.

13. 1-(4-Chlorphenoxy)-2-tert.butyl-2-hydroxy-3-fluor-3-methyl-3-(1H-1,2,4-triazol-1-yl)-propan gemäss Anspruch 1.

14. 1-(4-Methylphenoxy)-2-tert.butyl-2-hydroxy-3-fluor-3-methyl-3-(1H-1,2,4-triazol-1-yl)-propan gemäss Anspruch 1.

15. Fluorazolylketone der Formel V

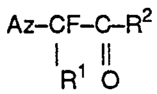

(V)

worin Az, $R^1$ und $R^2$ die unter Formel I im Anspruch 1 gegebene Bedeutung haben.

16. Fluorazolylmethyloxirane der Formel II

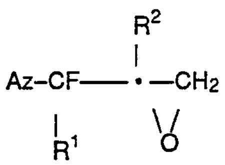

(II)

worin Az, $R^1$ und $R^2$ die unter Formel I im Anspruch 1 gegebene Bedeutung haben.

17. Verfahren zur Herstellung der Verbindungen der Formel I, dadurch gekennzeichnet, dass man ein Fluorazolylmethyloxiran der Formel II

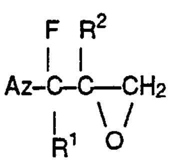

(II),

worin $R^1$, $R^2$ und Az die unter Formel I angegebene Bedeutung haben, in Gegenwart einer Base in einem inerten Lösungsmittel mit einem Alkohol oder Thioalkohol der Formel III

$$H-X-R^5 \quad (III),$$

worin X und $R^5$ die unter Formel I gegebene Bedeutung haben, umsetzt und das erhaltene Produkt der Formel Ia

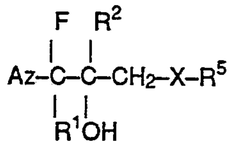

(Ia)

gewünschtenfalls durch Umsetzung mit einem Verätherungs- oder Veresterungsmittel der Formel IV

$Y - R^3$ (IV),

worin $R_3$ die unter Formel I gegebene Bedeutung hat und Y ein Halogenatom oder einen organischen oder anorganischen Säurerest bedeutet, veräthert oder verestert.

18. Verfahren nach Anspruch 17, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

19. Mittel zur Bekämpfung oder Verhütung eines Befalls durch Mikroorganismen und/oder zur Regulierung des Pflanzenwachstums, dadurch gekennzeichnet, dass es neben Träger- und/oder anderen Zuschlagstoffen als Wirkstoff mindestens eine Verbindung der Formel I gemäss Anspruch 1 enthält.

20. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

21. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

22. Verfahren gemäss Anspruch 20, dadurch gekennzeichnet, dass es sich bei den Mikroorganismen um phytopathogene Pilze handelt.

23. Verfahren gemäss Anspruch 20, zur Wuchshemmung im Sinne einer Erhöhung der Knickfestigkeit und Halmverkürzung bei Getreidesorten.

24. Verwendung gemäss Anspruch 20, dadurch gekennzeichnet, dass es sich bei den Getreidesorten um Hafer, Weizen, Gerste oder Roggen handelt.

25. Verfahren gemäss Anspruch 20 zur Wuchshemmung bei Gräsern und Unkräutern.

26. Verfahren gemäss Anspruch 20 zur Wuchsregulierung bei Leguminosen im Sinne einer Ertragssteigerung.

27. Verfahren gemäss Anspruch 26, dadurch gekennzeichnet, dass es sich um Soja handelt.

28. Verfahren gemäss Anspruch 20 zur Wuchshemmung von Bodenbedeckerpflanzen.

29. Verfahren gemäss Anspruch 20 zur Wuchshemmung und zur Wuchsregulierung von Baumwollpflanzen im Sinne einer Ertragssteigerung.

30. Verfahren gemäss Anspruch 20 zur Wuchsregulierung bei Getreidepflanzen im Sinne einer Ertragssteigerung.

**Patentansprüche** für den Vertragsstaat AT

1. Mittel zur Bekämpfung oder Verhütung eines Befalls durch Mikroorganismen und/oder zur Regulierung des Pflanzenwachstums, dadurch gekennzeichnet, dass es neben Träger- und/oder anderen Zuschlagstoffen als Wirkstoff mindestens ein Fluorazolylpropan-Derivat der Formel I

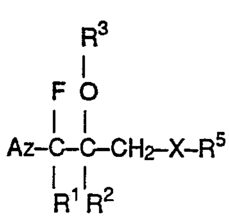

(I)

enthält, worin

Az für Imidazolyl oder 1,2,4-Triazolyl steht,
$R^1$ für Wasserstoff, für gegebenenfalls durch Halogen oder Cyan substituiertes $C_1$-$C_6$-Alkyl oder für gegebenenfalls durch Halogen $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiertes $C_1$-$C_3$-Phenylalkyl steht,
$R^2$ gegebenenfalls durch $C_1$-$C_3$-Alkoxy, Phenyl oder $C_{18}$-$C_3$-Phenylalkyl substituiertes $C_1$-$C_{10}$-Alkyl, bedeuten, wobei die Phenylkerne ihrerseits gegebenenfalls durch Halogen, $C_1$-$C_3$-Alkyl, $C_1$-$C_3$-Alkoxy, $C_1$-$C_3$-Halogenalkyl, Nitro, Cyan, Carboxyl oder $C_1$-$C_6$-Alkoxycarbonyl substituiert sein können,
$R^3$ für Wasserstoff, $C_1$-$C_6$-Alkyl, $C_1$-$C_6$-Alkylcarbonyl oder gegebenenfalls durch Halogen, $C_1$-$C_3$-Alkoxy, $C_1$-$C_3$-Alkyl, $C_1$-$C_3$-Halogenalkyl, Cyan, Carboxyl oder $C_1$-$C_6$-Alkoxycarbonyl substituiertes $C_1$-$C_3$-Phenylalkyl steht,
$R^5$ einen unsubstituierten oder ein- oder mehrfach substituierten Rest, steht, ausgewählt aus der Reihe $C_1$-$C_8$-Alkyl, $C_3$-$C_8$-Cycloalkyl, $C_3$-$C_6$-Alkenyl, $C_3$-$C_6$-Alkinyl, Phenyl, Naphthyl, Biphenyl, Benzylphenyl, Benzyloxyphenyl, Phenoxyphenyl und Aralkyl wobei die Substituenten aus der Reihe Halogen, Cyano, $C_1$-$C_3$-Alkyl, $C_1$-$C_3$-Alkoxy, $C_1$-$C_5$-Haloalkyl, $C_1$-$C_3$-Alkylthio, $C_1$-$C_3$-Haloalkylthio, $C_1$-$C_3$-Haloalkyl, Nitro und/oder Rhodano ausgewählt sind; und
X Sauerstoff oder Schwefel bedeuten; unter Einschluss der Säureadditionsalze quaternären Azoliumsalze und Metallkomplex.

2. Mittel gemäss Anspruch 1, dadurch gekennzeichnet, dass Az für 1,2,4-Triazolyl steht.

3. Mittel gemäss Anspruch 1, dadurch gekennzeichnet, dass $R^2$ $C_1$-$C_6$-Alkyl oder gegebenenfalls durch Halogen substituiertes Benzyl bedeutet.

4. Mittel gemäss Anspruch 3, dadurch gekennzeichnet, dass $R^2$ für tert.-Butyl oder i-Propyl steht.

5. Mittel gemäss Anspruch 1, dadurch gekennzeichnet, dass $R^1$ und $R^3$ Wasserstoff oder $C_1$-$C_4$-Alkyl bedeuten.

6. Mittel gemäss Anspruch 1, dadurch gekennzeichnet, dass $R^5$ für durch Halogen substituiertes Phenyl steht.

7. Mittel gemäss Anspruch 1, dadurch gekennzeichnet, dass $R^5$ für in 4-Stellung durch Halogen substituiertes Phenyl steht.

8. Mittel gemäss Anspruch 1, dadurch gekennzeichnet, dass X Sauerstoff ist.

9. Mittel gemäss Anspruch 1, dadurch gekennzeichnet, dass Az für 1,2,4-Triazolyl, $R^1$ und $R^3$ für Wasserstoff oder $C_1$-$C_4$-Alkyl, $R^2$ für tert.-Butyl oder i-Propyl, $R^5$ für in 4-Stellung durch Halogen substituiertes Phenyl und X für Sauerstoff stehen.

10. Mittel gemäss Anspruch 1, dadurch gekennzeichnet, dass es mindestens einen Wirkstoff enthält, ausgewählt aus der Gruppe

1-(4-Fluorphenoxy)-2-tert.butyl-2-hydroxy-3-fluor-3-(1H-1,2,4-triazol-1-yl)propan,
1-(4-Fluorphenoxy)-2-tert.butyl-2-hydroxy-3-fluor-3-methyl-3-(1H-1,2,4-triazol-1-yl)-propan
1-(4-Chlorphenoxy)-2-tert.butyl-2-hydroxy-3-fluor-3-(1H-1,2,4-triazol-1-yl)-propan,
1-(4-Chlorphenoxy)-2-tert.butyl-2-hydroxy-3-fluor-3-methyl-3-(1H-1,2,4-triazol-1-yl)-propan und
1-(4-Methylphenoxy)-2-tert.butyl-2-hydroxy-3-fluor-3-methyl-3-(1H-1,2,4-triazol-1-yl)-propan.

11. Verfahren zur Herstellung der Fluorazolylketone der Formel V

```
Az–CF–C–R²
    |  ||
    R¹ O                (V)
```

worin Az, $R^1$ und $R^2$ die unter Formel I im Anspruch 1 gegebene Bedeutung haben, dadurch gekennzeichnet, dass man die α-Halogenketone der Formel VI

```
    F  R²
    |  |
Hal–C–C=O               (VI)
    |
    R¹
```

worin $R^1$ und $R^2$ die unter Formel I gegebene Bedeutung haben und Hal für Chlor oder Brom steht, durch Umsetzung mit Azolen der Formel VII

H – Az (VII),

worin Az die unter Formel I gegebene Bedeutung hat, in Gegenwart einer Base umsetzt.

12. Verfahren zur Herstellung der Fluorazolylketone der Formel V gemäss Anspruch 11, dadurch gekennzeichnet, dass man ein Azolylmethylketon der Formel VIII

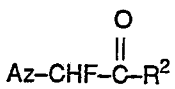
(VIII)

in Gegenwart einer Base mit einer Verbindung der Formel IX

$Z - R^1$ (IX)

worin $R^1$ die unter Formel I gegebene Bedeutung hat und Z für ein Halogenatom oder einen organischen oder anorganischen Säurerest steht, umsetzt.

13. Verfahren zur Herstellung der Fluorazolylmethyloxirane der Formel II

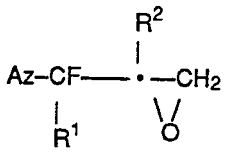
(II)

worin Az, $R^1$ und $R^2$ die unter Formel I im Anspruch 1 gegebene Bedeutung haben, dadurch gekennzeichnet, dass man ein Keton der Formel V

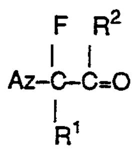
(V),

worin $R^1$, $R^2$ und Az die unter Formel I gegebenen Bedeutungen haben, mit Dimethylsulfoniummethylid, Dimethyloxosulfoniummethylid oder den entsprechenden Salzen in Gegenwart einer starken Base in einem polaren, reaktionsinerten Lösungsmittel umsetzt.

14. Verfahren zur Herstellung der Verbindungen der Formel I, dadurch gekennzeichnet, dass man ein Fluorazolylmethyloxiran der Formel II

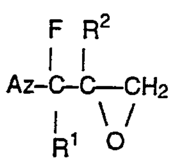
(II)

worin $R^1$, $R^2$ und Az die unter Formel I angegebene Bedeutung haben, in Gegenwart einer Base in einem inerten Lösungsmittel mit einem Alkohol oder Thioalkohol der Formel III

$H-X-R^5$ (III),

worin X und $R^5$ die unter Formel I gegebene Bedeutung haben, umsetzt und das erhaltene Produkt der Formel Ia

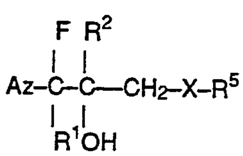
(Ia)

gewünschtenfalls durch Umsetzung mit einem Verätherungs- oder Veresterungsmittel der Formel IV

$Y - R^3$ (IV),

worin $R^3$ die unter Formel I gegebene Bedeutung hat und Y ein Halogenatom oder einen organischen oder anorganischen Säurerest bedeutet, veräthert oder verestert.

15. Verfahren nach Anspruch 14, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

16. Verfahren zur Bekämpfung oder Verhütung eines Befalls von Kulturpflanzen durch phytophatogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

17. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

18. Verfahren gemäss Anspruch 16, dadurch gekennzeichnet, dass es sich bei den Mikroorganismen um phytopathogene Pilze handelt.

19. Verfahren gemäss Anspruch 16 zur Wuchshemmung im Sinne einer Erhöhung der Knickfestigkeit und Halmverkürzung bei Getreidesorten.

20. Verwendung gemäss Anspruch 16, dadurch gekennzeichnet, dass es sich bei den Getreidesorten um Hafer, Weizen, Gerste oder Roggen handelt.

21. Verfahren gemäss Anspruch 16 zur Wuchshemmung bei Gräsern und Unkräutern.

22. Verfahren gemäss Anspruch 16 zur Wuchsregulierung bei Leguminosen im Sinne einer Ertragssteigerung.

23. Verfahren gemäss Anspruch 22, dadurch gekennzeichnet, dass es sich um Soja handelt.

24. Verfahren gemäss Anspruch 16 zur Wuchshemmung von Bodenbedeckerpflanzen.

25. Verfahren gemäss Anspruch 16 zur Wuchshemmung und zur Wuchsregulierung von Baumwollpflanzen im Sinne einer Ertragssteigerung.

26. Verfahren gemäss Anspruch 16 zur Wuchsregulierung bei Getreidepflanzen im Sinne einer Ertragssteigerung.

**Claims** for the Contracting States: BE, CH, LI, DE, FR, IT, NL, SE, GB

1. Fluoroazolylpropane derivatives of the formula I

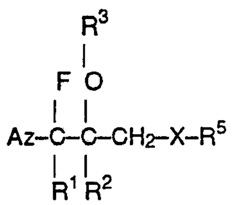

(I)

wherein Az is imidazolyl or 1,2,4-triazolyl,

$R^1$ is hydrogen, $C_1$-$C_6$-alkyl which is unsubstituted or substituted by halogen or by cyano, or $C_1$-$C_3$-phenylalkyl which is unsubstituted or substituted by halogen, $C_1$-$C_3$-alkyl, $C_1$-$C_3$-alkoxy, $C_1$-$C_3$-halogenoalkyl, nitro, cyano, carboxy or by $C_1$-$C_6$-alkoxycarbonyl,
$R^2$ is $C_1$-$C_{10}$-alkyl which is unsubstituted or substituted by $C_1$-$C_3$-alkoxy, phenyl or by $C_1$-$C_3$-phenylalkyl, it being possible for the phenyl nuclei in turn to be unsubstituted or substituted by halogen, $C_1$-$C_3$-alkyl, $C_1$-$C_3$-alkoxy, $C_1$-$C_3$-halogenoalkyl, nitro, cyano, carboxy or by $C_1$-$C_6$-alkoxycarbonyl,
$R^3$ is hydrogen, $C_1$-$C_6$-alkyl, $C_1$-$C_6$-alkylcarbonyl, or is $C_1$-$C_3$-phenylalkyl which is unsubstituted or substituted by halogen, $C_1$-$C_3$-alkoxy, $C_1$-$C_3$-alkyl, $C_1$-$C_3$-halogenoalkyl, cyano, carboxy or by $C_1$-$C_6$-alkoxycarbonyl,
$R^5$ is an unsubstituted or monosubstituted or polysubstituted radical selected from the series consisting of $C_1$-$C_8$-alkyl, $C_3$-$C_8$-cycloalkyl, $C_3$-$C_6$-alkenyl, $C_3$-$C_6$-alkynyl, phenyl, naphthyl, biphenyl, benzylphenyl, benzyloxyphenyl, phenoxyphenyl and aralkyl, the substituents being selected from the series consisting of halogen, cyano, $C_1$-$C_3$-alkyl, $C_1$-$C_5$-alkoxy, $C_1$-$C_5$-halogenoalkyl, $C_1$-$C_3$-alkylthio, $C_1$-$C_3$-halogenoalkylthio, $C_1$-$C_3$-halogenoalkyl, nitro and/or thiocyano, and
X is oxygen or sulfur; including the acid addition salts, quaternary azolium salts and metal complexes.

2. Compounds according to claim 1, wherein Az is 1,2,4-triazolyl.

3. Compounds according to claim 1, wherein $R^2$ is $C_1$-$C_6$-alkyl or is benzyl which is unsubstituted or substituted by halogen.

4. Compounds according to claim 3, wherein $R^2$ is tert.-butyl or isopropyl.

5. Compounds according to claim 1, wherein $R^1$ and $R^3$ are hydrogen or $C_1$-$C_4$-alkyl.

6. Compounds according to claim 1, wherein $R^5$ is phenyl which is substituted by halogen.

7. Compounds according to claim 1, wherein $R^5$ is phenyl which is substituted in the 4-position by halogen.

8. Compounds according to claim 1, wherein X is oxygen.

9. Compounds according to claim 1, wherein Az is 1,2,4-triazolyl, $R^1$ and $R^3$ are hydrogen or $C_1$-$C_4$-alkyl, $R^2$ is tert.-butyl or isopropyl, $R^5$ is phenyl which is substituted in the 4-position by halogen and X is oxygen.

10. 1-(4-fluorophenoxy)-2-tert.-butyl-2-hydroxy-3-fluoro-3-(1H-1,2,4-triazol-1-yl)-propane according to claim 1.

11. 1-(4-fluorophenoxy)-2-tert.-butyl-2-hydroxy-3-fluoro-3-methyl-3-(1H-1,2,4-triazol-1-yl)-propane according to claim 1.

12. 1-(4-chlorophenoxy)-2-tert.-butyl-2-hydroxy-3-fluoro-3-(1H-1,2,4-triazol-1-yl)-propane according to claim 1.

13. 1-(4-chlorophenoxy)-2-tert.-butyl-2-hydroxy-3-fluoro-3-methyl-3-(1H-1,2,4-triazol-1-yl)-propane according to claim 1.

14. 1-(4-methylphenoxy)-2-tert.-butyl-2-hydroxy-3-fluoro-3-methyl-3-(1H-1,2,4-triazol-1-yl)-propane according to claim 1.

15. Fluoroazolyl ketones of the formula V

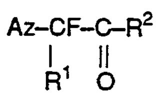

(V)

in which Az, $R^1$ and $R^2$ are as defined under formula I in claim 1.

16. Fluoroazolylmethyloxiranes of the formula II

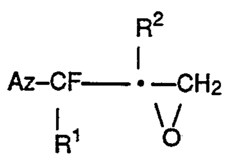

(II)

in which Az, $R^1$ and $R^2$ are as defined under formula I in claim 1.

17. A process for the preparation of the compounds of the formula I, which comprises reacting a fluoroazolylmethyloxirane of the formula II

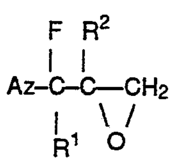

(II),

in which $R^1$, $R^2$ and Az are as defined under formula I, in the presence of a base and in an inert solvent, with an alcohol or thioalcohol of the formula III

$H–X–R^5$ (III)

in which X and $R^5$ are as defined under formula I, and, if desired, etherifying or esterifying the resulting product of the formula Ia

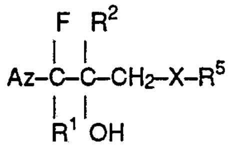

(Ia)

by reacting it with an etherifying or esterifying agent of the formula IV

$$Y - R^3 \quad \text{(IV)}$$

in which $R^3$ is as defined under formula I and Y is a halogen atom or an organic or inorganic acid radical.

18. A process according to claim 17, wherein the solvent employed is N,N-dimethylformamide, N,N-dimethylacetamide, dimethyl sulfoxide, acetonitrile or benzonitrile or a mixture of these solvents with one another or together with other conventional organic solvents which are inert towards the reaction.

19. A composition for controlling or preventing attack by microorganisms and/or for regulating plant growth, which, in addition to carriers and/or other adjuvants, contains at least one compound of the formula I according to claim 1 as the active substance.

20. A process for controlling or preventing attack on crop plants by phytopathogenic microorganisms and/or for regulating plant growth, wherein a compound of the formula I, defined in accordance with claim 1, is applied to the plant or to its locus in an effective amount.

21. Use of compounds of the formula I according to claim 1 for controlling and/or for prophylactically preventing attack by microorganisms and/or for regulating plant growth.

22. A process according to claim 20, wherein the microorganisms are phytopathogenic fungi.

23. A process according to claim 20 for inhibiting growth for the purposes of increasing resistance to lodging and of shortening the stems of cereal species.

24. Use according to claim 20, wherein the species of cereal are oats, wheat, barley or rye.

25. A process according to claim 20 for inhibiting growth in grasses and weeds.

26. A process according to claim 20 for regulating the growth of leguminosae for the purposes of increasing the yield.

27. A process according to claim 26, which relates to soybeans.

28. A process according to claim 20 for inhibiting the growth of ground-cover plants.

29. A process according to claim 20 for inhibiting the growth and regulating the growth of cotton plants for the purposes of increasing the yield.

30. A process according to claim 20 for regulating the growth of cereal plants for the purposes of increasing the yield.

**Claims** for the Contracting State: AT

1. A composition for controlling or preventing attack by microorganisms and/or for regulating plant growth, which, in addition to carriers and/or other adjuvants, contains as the active substance at least one fluoroazolylpropane derivative of the formula I

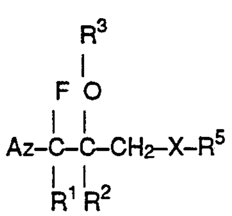

(I)

wherein Az is imidazolyl or 1,2,4-triazolyl,

$R^1$ is hydrogen, $C_1$-$C_6$-alkyl which is unsubstituted or substituted by halogen or by cyano, or $C_1$-$C_3$-phenylalkyl which is unsubstituted or substituted by halogen, $C_1$-$C_3$-alkyl, $C_1$-$C_3$-alkoxy, $C_1$-$C_3$-halogenoalkyl, nitro, cyano, carboxy or by $C_1$-$C_6$-alkoxycarbonyl,

$R^2$ is $C_1$-$C_{10}$-alkyl which is unsubstituted or substituted by $C_1$-$C_3$-alkoxy, phenyl or by $C_1$-$C_3$-phenylalkyl, it being possible for the phenyl nuclei in turn to be unsubstituted or substituted by halogen, $C_1$-$C_3$-alkyl, $C_1$-$C_3$-alkoxy, $C_1$-$C_3$-halogenoalkyl, nitro, cyano, carboxy or by $C_1$-$C_6$-alkoxycarbonyl,

$R^3$ is hydrogen, $C_1$-$C_6$-alkyl, $C_1$-$C_6$-alkylcarbonyl, or is $C_1$-$C_3$-phenylalkyl which is unsubstituted or substituted by halogen, $C_1$-$C_3$-alkoxy, $C_1$-$C_3$-alkyl, $C_1$-$C_3$-halogenoalkyl, cyano, carboxy or by $C_1$-$C_6$-alkoxycarbonyl,

$R^5$ is an unsubstituted or monosubstituted or polysubstituted radical selected from the series consisting of $C_1$-$C_8$-alkyl, $C_3$-$C_8$-cycloalkyl, $C_3$-$C_6$-alkenyl, $C_3$-$C_6$-alkynyl, phenyl, naphthyl, biphenyl, benzylphenyl, benzyloxyphenyl, phenoxyphenyl and aralkyl, the substituents being selected from the series consisting of halogen, cyano, $C_1$-$C_3$-alkyl, $C_1$-$C_5$-alkoxy, $C_1$-$C_5$-halogenoalkyl, $C_1$-$C_3$-alkylthio, $C_1$-$C_3$-halogenoalkylthio, $C_1$-$C_3$-halogenoalkyl, nitro and/or thiocyano, and

X is oxygen or sulfur; including the acid addition salts, quaternary azolium salts and metal complexes.

2. A composition according to claim 1, wherein Az is 1,2,4-triazolyl.

3. A composition according to claim 1, wherein $R^2$ is $C_1$-$C_6$-alkyl or is benzyl which is unsubstituted or substituted by halogen.

4. A composition according to claim 3, wherein $R^2$ is tert.-butyl or isopropyl.

5. A composition according to claim 1, wherein $R^1$ and $R^3$ are hydrogen or $C_1$-$C_4$-alkyl.

6. A composition according to claim 1, wherein $R^5$ is phenyl which is substituted by halogen.

7. A composition according to claim 1, wherein $R^5$ is phenyl which is substituted in the 4-position by halogen.

8. A composition according to claim 1, wherein X is oxygen.

9. A composition according to claim 1, wherein Az is 1,2,4-triazolyl, $R^1$ and $R^3$ are hydrogen or $C_1$-$C_4$-alkyl, $R^2$ is tert.-butyl or isopropyl, $R^5$ is phenyl which is substituted in the 4-position by halogen and X is oxygen.

10. A composition according to claim 1, which contains at least one active substance selected from the group consisting of

1-( 4-fluorophenoxy )-2-tert.-butyl-2-hydroxy-3-fluoro-3-( 1H-1,2,4-triazol-1-yl )-propane,
1-( 4-fluorophenoxy )-2-tert.-butyl-2-hydroxy-3-fluoro-3-methyl-3-( 1H-1,2,4-triazol-1-yl )-propane,
1-( 4-chlorophenoxy )-2-tert.-butyl-2-hydroxy-3-fluoro-3-( 1H-1,2,4-triazol-1-yl )-propane,
1-( 4-chlorophenoxy )-2-tert.-butyl-2-hydroxy-3-fluoro-3-methyl-3-( 1H-1,2,4-triazol-1-yl )-propane and
1-( 4-methylphenoxy )-2-tert.-butyl-2-hydroxy-3-fluoro-3-methyl-3-( 1H-1,2,4-triazol-1-yl )-propane.

11. A process for the preparation of the fluoroazolyl ketones of the formula V

```
Az–CF–C–R²
    |  ||
    R¹ O
```
(V)

in which Az, $R^1$ and $R^2$ are as defined under formula I in claim 1, which comprises treating the α-halogenoketones of the formula VI

```
    F  R²
    |  |
Hal–C–C=O
    |
    R¹
```
(VI)

in which $R^1$ and $R^2$ are as defined under formula I and Hal is chlorine or bromine, with azoles of the formula VII

H – Az (VII)

in which Az is as defined under formula I, in the presence of a base.

12. A process for the preparation of the fluoroazolyl ketones of the formula V according to claim 11, which comprises

reacting an azolyl methyl ketone of the formula VIII

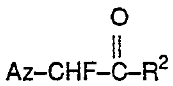

(VIII)

in the presence of a base with a compound of the formula IX

$Z - R^1$ (IX)

in which $R^1$ is as defined under formula I and Z is a halogen atom or an organic or inorganic acid radical.

13. A process for the preparation of the fluoroazolylmethyloxiranes of the formula II

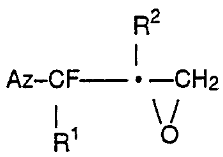

(II)

in which Az, $R^1$ and $R^2$ are as defined under formula I in claim 1, which comprises reacting a ketone of the formula V

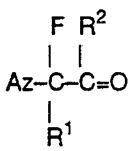

(V)

in which $R^1$, $R^2$ and Az are as defined under formula I, with dimethylsulfonium methylide, dimethyloxosulfonium methylide or the corresponding salts in the presence of a strong base and in a polar solvent which is inert towards the reaction.

14. A process for the preparation of the compounds of the formula I, which comprises reacting a fluoroazolylmethyloxirane of the formula II

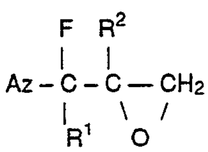

(II)

in which $R^1$, $R^2$ and Az are as defined under formula I, in the presence of a base and in an inert solvent, with an alcohol or thioalcohol of the formula III

$H - X - R^5$ (III)

in which X and $R^5$ are as defined under formula I, and, if desired, etherifying or esterifying the resulting product of the formula Ia

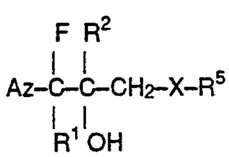

(Ia)

by reacting it with an etherifying or esterifying agent of the formula IV

$Y - R^3$ (IV)

in which $R^3$ is as defined under formula I and Y is a halogen atom or an organic or inorganic acid radical.

15. A process according to claim 14, wherein the solvent employed is N,N-dimethylformamide, N,N-dimethylacetamide, dimethyl sulfoxide, acetonitrile or benzonitrile or a mixture of these solvents with one another or together with other conventional organic solvents which are inert towards the reaction.

16. A process for controlling or preventing attack on crop plants by phytopathogenic microorganisms and/or for regulating plant growth, wherein a compound of the formula I, defined in accordance with claim 1, is applied to the plant or to its locus in an effective amount.

17. Use of compounds of the formula I according to claim 1 for controlling and/or for prophylactically preventing attack by microorganisms and/or for regulating plant growth.

18. A process according to claim 16, wherein the microorganisms are phytopathogenic fungi.

19. A process according to claim 16 for inhibiting growth for the purposes of increasing resistance to lodging and of shortening the stems of cereal species.

20. Use according to claim 16, wherein the species of cereal are oats, wheat, barley or rye.

21. A process according to claim 16 for inhibiting growth in grasses and weeds.

22. A process according to claim 16 for regulating the growth of leguminosae for the purposes of increasing the yield.

23. A process according to claim 22, which relates to soybeans.

24. A process according to claim 16 for inhibiting the growth of ground-cover plants.

25. A process according to claim 16 for inhibiting the growth and regulating the growth of cotton plants for the purposes of increasing the yield.

26. A process according to claim 16 for regulating the growth of cereal plants for the purposes of increasing the yield.

**Revendications** pour les Etats Contractants: BE, CH, LI, DE, FR, IT, NL, SE, GB

1. Dérivés du fluorazolylpropane répondant à la formule I

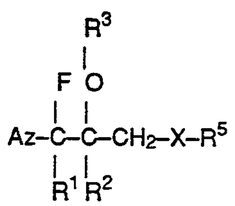

(I)

dans laquelle

Az représente un groupe imidazolyle ou 1,2,4-triazolyle,
$R^1$ représente l'hydrogène, un groupe alkyle en $C_1$-$C_6$ éventuellement substitué par des halogènes ou des groupes cyano ou un groupe phényl-alkyle en $C_1$-$C_3$ éventuellement substitué par des halogènes, des groupes alkyle en $C_1$-$C_3$, alcoxy en $C_1$-$C_3$, halogénoalkyle en $C_1$-$C_3$, nitro, cyano, carboxyle ou (alcoxy en $C_1$-$C_6$)-carbonyle,
$R^2$ représente un groupe alkyle en $C_1$-$C_{10}$ éventuellement substitué par des groupes alcoxy en $C_1$-$C_3$; phényle ou phényl-alkyle en $C_1$-$C_3$, les noyaux phényle pouvant eux-mêmes être éventuellement substitués par des halogènes, des groupes alkyle en $C_1$-$C_3$, alcoxy en $C_1$-$C_3$, halogénoalkyle en $C_1$-$C_3$, nitro, cyano, carboxyle ou (alcoxy en $C_1$-$C_6$)-carbonyle,
$R^3$ représente l'hydrogène, un groupe alkyle en $C_1$-$C_6$, (alkyle en $C_1$-$C_6$)-carbonyle ou phényl-alkyle en $C_1$-$C_3$ éventuellement substitué par des halogènes, des groupes alcoxy en $C_1$-$C_3$, alkyle en $C_1$-$C_3$, halogénoalkyle en $C_1$-$C_3$, cyano, carboxyle ou (alcoxy en $C_1$-$C_6$)-carbonyle,
$R^5$ représente un groupe non substitué ou mono- ou polysubstitué choisi parmi les groupes alkyle en $C_1$-$C_8$, cycloalkyle en $C_3$-$C_8$, alcényle en $C_3$-$C_6$, alcynyle en $C_3$-$C_6$, phényle, naphtyle, biphényle, benzylphényle, benzyloxyphényle, phénoxyphényle et aralkyle, les substituants étant choisis parmi les atomes d'halogènes, les groupes cyano, alkyle en $C_1$-$C_3$, alcoxy en $C_1$-$C_5$, halogénoalkyle en $C_1$-$C_5$, alkylthio en $C_1$-$C_3$, halogénoalkylthio en $C_1$-$C_3$, halogénoalkyle en $C_1$-$C_3$, nitro et/ou thiocyano; et
X représente l'oxygène ou le soufre; y compris les sels formés par addition avec des acides, les sels d'azolium quaternaires et les complexes métalliques.

2. Composés selon la revendication 1, caractérisés en ce que Az représente le groupe 1,2,4-triazolyle.

3. Composés selon la revendication 1, caractérisés en ce que $R^2$ représente un groupe alkyle en $C_1$-$C_6$ ou un groupe benzyle éventuellement substitué par des halogènes.

4. Composés selon la revendication 3, caractérisés en ce que $R^2$ représente un groupe, tert-butyle ou isopropyle.

5. Composés selon la revendication 1, caractérisés en ce que $R^1$ et $R^3$ représentent l'hydrogène ou des groupes alkyle en $C_1$-$C_4$.

6. Composés selon la revendication 1, caractérisés en ce que $R^5$ représente un groupe phényle substitué par des halogènes.

7. Composés selon la revendication 1, caractérisés en ce que $R^5$ représente un groupe phényle substitué en position 4 par un halogène.

8. Composés selon la revendication 1, caractérisés en ce que X représente l'oxygène.

9. Composés selon la revendication 1, caractérisés en ce que Az représente le groupe 1,2,4-triazolyle, $R^1$ et $R^3$ représentent l'hydrogène ou des groupes alkyle en $C_1$-$C_4$, $R^2$ représente un groupe tert-butyle ou isopropyle, $R^5$ représente un groupe phényle substitué en position 4 par un halogène et X représente l'oxygène.

10. Le 1-(4-fluorophénoxy)-2-tert-butyl-2-hydroxy-3-fluoro-3-(1H-1,2,4-triazole-1-yl)-propane selon la revendication 1.

11. Le 1-(4-fluorophénoxy)-2-tert-butyl-2-hydroxy-3-fluoro-3-méthyl-3-(1H-1,2,4-triazole-1-yl)-propane selon la revendication 1.

12. Le 1-(4-chlorophénoxy)-2-tert-butyl-2-hydroxy-3-fluoro-3-(1H-1,2,4-triazole-1-yl)-propane selon la revendication 1.

13. Le 1-(4-chlorophénoxy)-2-tert-butyl-2-hydroxy-3-fluoro-3-méthyl-3-(1H-1,2,4-triazole-1-yl)-propane selon la revendication 1.

14. Le 1-(4-méthylphénoxy)-2-tert-butyl-2-hydroxy-3-fluoro-3-méthyl-3-(1H-1,2,14-triazole-1-yl)-propane selon la revendication 1.

15. Fluorazolylcétones de formule V

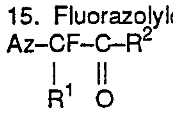

(V)

dans laquelle Az, $R^1$ et $R^2$ ont les significations indiquées dans la revendication 1 en référence à la formule I.

16. Fluorazolylméthyloxirannes de formule II

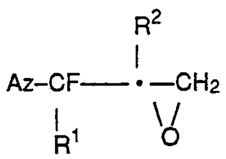

(II)

dans laquelle Az, $R^1$ et $R^2$ ont les significations indiquées dans la revendication 1 en référence à la formule I.

17. Procédé de préparation des composés de formule I, caractérisé en ce que l'on fait réagir un fluorazolylméthyloxiranne de formule II

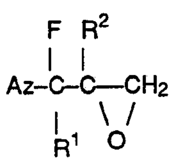

(II),

dans laquelle $R^1$, $R^2$ et Az ont les significations indiquées en référence à la formule I, en présence d'une base, dans un solvant inerte, avec un alcool ou thioalcool de formule III

$$H - X - R^5 \quad \text{(III)},$$

dans laquelle X et $R^5$ ont les significations indiquées en référence à la formule I, ce qui donne le composé de formule Ia

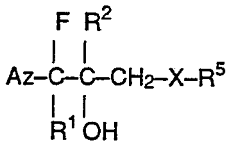

(Ia)

qu'on éthérifie ou qu'on estérifie si on le désire par réaction avec un agent éthérifiant ou estérifiant de formule IV

$$Y - R^3 \qquad (IV),$$

dans laquelle $R^3$ a les significations indiquées en référence à la formule I et Y représente un atome d'halogène ou un radical d'acide organique ou minéral.

18. Procédé selon la revendication 17, caractérisé en ce que l'on utilise en tant que solvant le N,N-diméthylformamide, le N,N-diméthylacétamide, le diméthylsulfoxyde, l'acétonitrile ou le benzonitrile ou un mélange de ces solvants entre eux ou avec d'autres solvants organiques usuels inertes dans la réaction.

19. Produit pour combattre ou prévenir une attaque par des microorganismes et/ou pour la régulation de la croissance des végétaux, caractérisé en ce qu'il contient en tant que substance active, avec des véhicules et/ou d'autres additifs, au moins un composé de formule I selon la revendication 1.

20. Procédé pour combattre ou prévenir une attaque de végétaux cultivés par des microorganismes phytopathogènes et/ou pour la régulation de la croissance des végétaux, caractérisé en ce que l'on applique sur la plante ou son habitat une quantité efficace d'un composé de formule I définie dans la revendication 1.

21. Utilisation des composés de formule I selon la revendication 1 pour la lutte et/ou la prévention d'une attaque par les microorganismes et/ou pour la régulation de la croissance des végétaux.

22. Procédé selon la revendication 1, caractérisé en ce que les microorganismes sont des mycètes phytopathogènes.

23. Procédé selon la revendication 20, pour l'inhibition de la croissance dans le sens d'une amélioration de la résistance à la verse et d'un raccourcissement de la tige chez les céréales.

24. Utilisation selon la revendication 20, caractérisée en ce que les céréales sont l'avoine, le blé, l'orge ou le seigle.

25. Procédé selon la revendication 20, pour l'inhibition de la croissance des graminées et des végétaux adventices.

26. Procédé selon la revendication 20, pour la régulation de la croissance des légumineuses dans le sens d'une augmentation de rendement.

27. Procédé selon la revendication 26, caractérisé en ce que la légumineuse est le soja.

28. Procédé selon la revendication 20, pour l'inhibition de la croissance des végétaux de couvertures de sol.

29. Procédé selon la revendication 20, pour l'inhibition de la croissance et pour la régulation de la croissance des plants de coton dans le sens d'une augmentation de rendement.

30. Procédé selon la revendication 20, pour la régulation de la croissance des céréales dans le sens d'une augmentation de rendement.

**Revendications** pour l'Etat Contractant: AT

1. Produit pour combattre ou prévenir une attaque par des microorganismes et/ou pour la régulation de la croissance des végétaux, caractérisé en ce qu'il contient en tant que substance active, avec des véhicules et/ou d'autres additifs, au moins un dérivé du fluorazolylpropane répondant à la formule I.

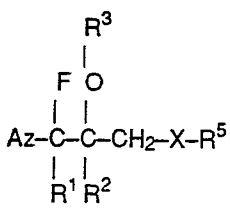

(I)

dans laquelle

Az représente un groupe imidazolyle ou 1,2,4-triazolyle,
$R^1$ représente l'hydrogène, un groupe alkyle en $C_1$-$C_6$ éventuellement substitué par des halogènes ou des groupes cyano ou un groupe phényl-alkyle en $C_1$-$C_3$ éventuellement substitué par des halogènes, des groupes alkyle en $C_1$-$C_3$, alcoxy en $C_1$-$C_3$, halogénoalkyle en $C_1$-$C_3$, nitro, cyano, carboxyle ou (alcoxy en $C_1$-$C_6$)-carbonyle,
$R^2$ représente un groupe alkyle en $C_1$-$C_{10}$ éventuellement substitué par des groupes alcoxy en $C_1$-$C_3$, phényle ou phényl-alkyle en $C_1$-$C_3$, les noyaux phényle pouvant eux-mêmes être éventuellement substitués par des halogènes, des groupes alkyle en $C_1$-$C_3$, alcoxy en $C_1$-$C_3$, halogénoalkyle en $C_1$-$C_3$, nitro, cyano, carboxyle ou (alcoxy en $C_1$-$C_6$)-carbonyle,
$R^3$ représente l'hydrogène, un groupe alkyle en $C_1$-$C_6$, (alkyle en $C_1$-$C_6$)-carbonyle ou un groupe phénylalkyle en $C_1$-$C_3$ éventuellement substitué par des halogènes, des groupes alcoxy en $C_1$-$C_3$, alkyle en $C_1$-$C_3$, halogénoalkyle en $C_1$-$C_3$, cyano, carboxyle ou (alcoxy en $C_1$-$C_6$)-carbonyle,
$R^5$ représente un groupe non substitué ou mono- ou polysubstitué choisi parmi les groupes alkyle en $C_1$-$C_8$, cycloalkyle en $C_3$-$C_8$, alcényle en $C_3$-$C_6$, alcynyle en $C_3$-$C_6$, phényle, naphtyle, biphényle, benzylphényle, benzyloxyphényle, phénoxyphényle et aralkyle, les substituants étant choisis parmi les halogènes, les groupes cyano, alkyle en $C_1$-$C_3$, alcoxy en $C_1$-$C_5$, halogénoalkyle en $C_1$-$C_5$, alkylthio en $C_1$-$C_3$, halogénoalkylthio en $C_1$-$C_3$, halogénoalkyle en $C_1$-$C_3$, nitro et/ou thiocyano; et
X représente l'oxygène ou le soufre; y compris les sels formés par addition avec des acides, sels d'azolium quaternaires et complexes métalliques.

2. Produit selon la revendication 1, caractérisé en ce que Az représente un groupe 1,2,4-triazolyle.

3. Produit selon la revendication 1, caractérisé en ce que $R^2$ représente un groupe alkyle en $C_1$-$C_6$ ou un groupe benzyle éventuellement substitué par des halogènes.

4. Produit selon la revendication 3, caractérisé en ce que $R^2$ représente un groupe tert-butyle ou isopropyle.

5. Produit selon la revendication 1, caractérisé en ce que $R^1$ et $R^3$ représentent l'hydrogène ou des groupes alkyle en $C_1$-$C_4$.

6. Produit selon la revendication 1, caractérisé en ce que $R^5$ représente un groupe phényle substitué par des halogènes.

7. Produit selon la revendication 1, caractérisé en ce que $R^5$ représente un groupe phényle substitué en position 4 par un halogène.

8. Produit selon la revendication 1, caractérisé en ce que X représente l'oxygène.

9. Produit selon la revendication 1, caractérisé en ce que Az représente un groupe 1,2,4-triazolyle, $R^1$ et $R^3$ représentent l'hydrogène ou des groupes alkyle en $C_1$-$C_4$, $R^2$ représente un groupe tert-butyle ou isopropyle, $R^5$ représente un groupe phényle substitué en position 4 par un halogène et X représente l'oxygène.

10. Produit selon la revendication 1, caractérisé en ce qu'il contient au moins une substance active choisie dans le groupe suivant:

1-( 4-fluorophénoxy )-2-tert-butyl-2-hydroxy-3-fluoro-3-( 1H-1,2,4-triazole-1-yl )-propane,
1-( 4-fluorophénoxy )-2-tert-butyl-2-hydroxy-3-fluoro-3-méthyl-3-( 1H-1,2,4-triazole-1-yl )-propane,
1-( 4-chlorophénoxy )-2-tert-butyl-2-hydroxy-3-fluoro-3-( 1H-1,2,4-triazole-1-yl )-propane,
1-( 4-chlorophénoxy )-2-tert-butyl-2-hydroxy-3-fluoro-3-méthyl-3-( 1H-1,2,4-triazole-1-yl )-propane et
1-( 4-méthylphénoxy )-2-tert-butyl-2-hydroxy-3-fluoro-3-méthyl-3-( 1H-1,2,4-triazole-1-yl )-propane.

11. Procédé de préparation des fluorazolylcétones de formule V

(V)

Az–CF–C–$R^2$
| ||
$R^1$ O

dans laquelle Az, $R^1$ et $R^2$ ont les significations indiquées dans la revendication 1 en référence à la formule I, caractérisé en ce que l'on fait réagir les alphahalogénocétones de formule VI

F $R^2$
| |
Hal–C–C=O (VI)
|
$R^1$

dans laquelle $R^1$ et $R^2$ ont les significations indiquées en référence à la formule I et Hal représente le chlore ou le brome, avec des azoles de formule VII

H – Az (VII)

dans laquelle Az a les significations indiquées en référence à la formule I, en présence d'une base.

12. Procédé de préparation des fluorazolylcétones de formule V selon la revendication 11, caractérisé en ce que l'on fait réagir une azolylméthylcétone de formule VIII

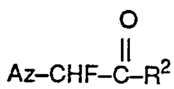

(VIII)

en présence d'une base, avec un composé de formule IX

Z – $R^1$ (IX)

dans laquelle $R^1$ a les significations indiquées en référence à la formule I et Z représente un atome d'halogène ou un radical d'acide organique ou minéral.

13. Procédé de préparation des fluorazolylméthyloxirannes de formule II

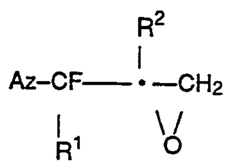

(II)

dans laquelle Az, $R^1$ et $R^2$ ont les significations indiquées dans la revendication 1 en référence à la formule I, caractérisé en ce que l'on fait réagir une cétone de formule V

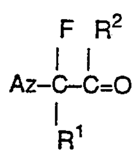

(V),

dans laquelle $R^1$, $R^2$ et Az ont les significations indiquées en référence à la formule I, avec le méthylure de diméthylsulfonium, le méthylure de diméthyloxosulfonium ou les sels correspondants en présence d'une base forte dans un solvant polaire inerte dans la réaction.

14. Procédé de préparation des composés de formule I, caractérisé en ce que l'on fait réagir un fluorazolylméthyloxiranne de formule II

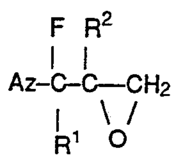

(II),

dans laquelle $R^1$, $R^2$ et Az ont les significations indiquées en référence à la formule I, en présence d'une base, dans un solvant inerte, avec un alcool ou thioalcool de formule III

H – X– $R^5$ (III)

dans laquelle X et $R^5$ ont les significations indiquées en référence à la formule I,
ce qui donne un composé de formule Ia

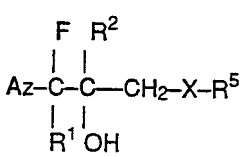

(Ia)

qu'on éthérifie ou estérifie si on le désire par réaction avec un agent éthérifiant ou estérifiant de formule IV

$$Y - R^3 \qquad (IV)$$

dans laquelle $R^3$ a les significations indiquées en référence à la formule I et Y représente un atome d'halogène ou un radical d'acide organique ou minéral.

15. Procédé selon la revendication 14, caractérisé en ce que l'on utilise en tant que solvant le N,N-diméthylformamide, le N,N-diméthylacétamide, le diméthylsulfoxyde, l'acétonitrile ou le benzonitrile ou un mélange de ces solvants entre eux ou avec d'autres solvants organiques usuels inertes dans la réaction.

16. Procédé pour combattre ou prévenir une attaque de végétaux cultivés par des microorganismes phytopathogènes et/ou pour la régulation de la croissance des végétaux, caractérisé en ce que l'on applique sur la plante ou son habitat une quantité efficace d'un composé de formule I définie dans la revendication 1.

17. Utilisation des composés de formule I selon la revendication 1 pour combattre et/ou prévenir une attaque par des microorganismes et/ou pour la régulation de la croissance des végétaux.

18. Procédé selon la revendication 16, caractérisé en ce que les microorganismes sont des mycètes phytopathogènes.

19. Procédé selon la revendication 16, pour l'inhibition de la croissance dans le sens d'une augmentation de la résistance à la verse et d'un raccourcissement de la tige chez les céréales.

20. Utilisation selon la revendication 16, caractérisée en ce que les céréales consistent en avoine blé, orge ou seigle.

21. Procédé selon la revendication 16, pour l'inhibition de la croissance des graminées et des végétaux adventices.

22. Procédé selon la revendication 16, pour la régulation de la croissance des légumineuses dans le sens d'une augmentation de rendement.

23. Procédé selon la revendication 22, caractérisé en ce que les légumineuses consistent en soja.

24. Procédé selon la revendication 16, pour l'inhibition de la croissance des végétaux de couvertures de sol.

25. Procédé selon la revendication 16, pour l'inhibition de la croissance et pour la régulation de la croissance des plants de coton dans le sens d'une augmentation de rendement.

26. Procédé selon la revendication 16, pour la régulation de la croissance des céréales dans le sens d'une augmentation de rendement.